(19)

**Europäisches Patentamt**
**European Patent Office**
**Office européen des brevets**

(11) **EP 4 653 467 A1**

(12) **EUROPEAN PATENT APPLICATION**
published in accordance with Art. 153(4) EPC

(43) Date of publication:
   **26.11.2025 Bulletin 2025/48**

(21) Application number: **24744212.2**

(22) Date of filing: **15.01.2024**

(51) International Patent Classification (IPC):
   *C07K 19/00* (2006.01)    *C12N 15/86* (2006.01)
   *A61P 35/00* (2006.01)    *A61P 15/02* (2006.01)

(52) Cooperative Patent Classification (CPC):
   **A61K 39/00; A61P 15/02; A61P 35/00;
   A61P 35/02; C07K 19/00; C12N 5/10; C12N 15/62;
   C12N 15/86; C12N 15/867**

(86) International application number:
   **PCT/CN2024/072334**

(87) International publication number:
   **WO 2024/153036 (25.07.2024 Gazette 2024/30)**

(84) Designated Contracting States:
   **AL AT BE BG CH CY CZ DE DK EE ES FI FR GB
   GR HR HU IE IS IT LI LT LU LV MC ME MK MT NL
   NO PL PT RO RS SE SI SK SM TR**
   Designated Extension States:
   **BA**
   Designated Validation States:
   **GE KH MA MD TN**

(30) Priority: **19.01.2023 CN 202310057155**

(71) Applicant: **Wuhan Zhaozhi Biotechnology Co.,
   Ltd.**
   **Wuhan, Hubei 430206 (CN)**

(72) Inventors:
   • **LI, Weiyong**
    **Wuhan, Hubei 430206 (CN)**

   • **GU, Chaojiang**
    **Wuhan, Hubei 430206 (CN)**
   • **ZHOU, Yuan**
    **Wuhan, Hubei 430206 (CN)**

(74) Representative: **Henderson, Helen Lee**
   **Withers & Rogers LLP**
   **2 London Bridge**
   **London SE1 9RA (GB)**

Remarks:
   The complete document including Reference
   Table(s) and the Sequence Listing(s) can be
   downloaded from the EPO website

(54) **CAR MOLECULE, CELL OR EXOSOME CONTAINING SAME, AND USE THEREOF**

(57)    A CAR molecule, a cell or exosome containing same, and the use thereof. The CAR molecule is a CAR molecule targeting CD19 and/or a CAR molecule targeting CD22. Also disclosed are a nucleic acid encoding the CAR molecule, a recombinant expression vector containing the nucleic acid, and a pharmaceutical composition containing the CAR molecule or the cell or exosome containing same. A CAR-T cell containing the CAR molecule has higher therapeutic effectiveness and therapeutic safety in clinical treatment; and the exosome provided has a better targeting performance, can achieve better tumor penetration, and is expected to become a new direction for treating tumors with CAR-T cell therapy.

FIG. 1

EP 4 653 467 A1

**Description**

The present application claims priority to Chinese patent application 2023100571558 filed on January 19, 2023. The contents of the Chinese patent application are incorporated herein by reference in their entirety.

TECHNICAL FIELD

The present disclosure belongs to the field of biotechnology, and specifically relates to a CAR molecule, a cell or exosome containing same, and the use thereof.

BACKGROUND

Cancer is one of the most lethal diseases worldwide. The primary treatment methods for tumors include surgical resection, chemotherapy, radiotherapy, and immunotherapy. Balancing the toxicity of drugs to tumors and the precise delivery of drugs to tumor sites can better exert the therapeutic effects while avoiding toxic side effects on normal cells and tissues.

The theoretical basis of tumor immunotherapy lies in that the immune system has the ability to recognize tumor-associated antigens and regulate the body to attack on tumor cells (highly specific cytolysis). The biological process is highly complex and remains under investigation. In the 1990s, several research groups have identified tumor antigens that can be recognized by T lymphocytes in a major histocompatibility complex (MHC)-dependent manner.

Tumor immunotherapy is generally divided into two categories: non-specific immunotherapy and specific immunotherapy. Non-specific immunotherapy mainly includes cytokines and toxins such as interleukin-2 (IL-2), interferon $\alpha$ (IFN-$\alpha$), tumor necrosis factor (TNF-$\alpha$), and BCG, as well as adoptive cellular immunotherapy. Specific immunotherapy primarily involves tumor vaccines.

A chimeric antigen receptor (CAR) is formed by the fusion of an extracellular antigen-targeting binding domain, a transmembrane domain, and an intracellular T cell activation signaling domain. The introduction of CAR into T cells by exogenous genes followed by the expression of CAR molecules in effector T cells confers the cells with specific antigen recognition capability. CAR-T cell therapy is a method of expanding these cells *in vitro* on a large scale and then infusing them into patients for treatment. Currently, CAR-T therapy has achieved remarkably encouraging success in B cell-derived tumors and leukemia.

Compared to traditional adoptive immunotherapy, CAR-T therapy primarily offers the following two advantages: first, CAR-T cells directly bind to antigen epitopes in a major histocompatibility complex (MHC)-independent manner, enabling immune cells to specifically recognize and kill tumors; second, CAR-T cells can highly express receptor molecules on the cell membrane that specifically recognize natural antigens, without relying on antigen-presenting cells to process natural antigens, and the target antigen can be either protein-based, carbohydrate-based, or lipid-based.

CARs consist of three modules: an extracellular target-binding module (ligand binding domain); a transmembrane domain for anchoring the cell membrane; and an intracellular signaling module (signaling domains and cosignaling domains) for transmitting activation signals. The target-binding module is typically based on a single-chain variable fragment (scFv), in which the VH and VL sequences are linked; the intracellular signaling module typically consists of intracellular segments of signaling molecules such as CD3$\zeta$ chain and co-stimulatory molecules such as CD28 involved in T cell activation, thereby transmitting TCR (T cell receptor) activation signals into the cells. The CAR-transduced T cells exhibit antigen specificity and cytotoxicity of effector T cells. When the tumor-associated antigen itself serves as a CAR-T cell receptor, the extracellular domain of CAR-T cells is mostly its ligand or polypeptide, thereby conferring tumor-associated antigen specificity to CAR-T cells. Upon recognizing specific tumor-associated antigens, CARs trigger specific activation of CAR-T cells under the combined action of intracellular T cell activation signaling motifs (CD3$\zeta$) and co-stimulatory signaling motifs in tandem, which is manifested as extensive proliferation of CAR-T cells, secretion of functional cytokines, and CAR-T cell-dependent killing effects.

Currently, CAR-T technology has evolved to the fourth generation, which is primarily classified based on the number of signaling motifs in tandem in the intracellular domain of the CAR molecule. Specifically, a CAR with one intracellular signaling motif is defined as the first-generation CAR. Since the first-generation CAR molecule contains only a single CD3$\zeta$ in the intracellular signaling domain, cell activation signals are primarily transmitted through its immunoreceptor tyrosine-based activation motif (ITAM) and activate related signaling pathways. Clinical studies on early tumor treatment have shown that CAR-T cell therapy exhibits only limited therapeutic efficacy in most clinical cases, as the infused cells cannot persist long-term *in vivo.*

Since the *in vivo* half-life of adoptively transferred CAR-T cells in clinical cases is closely related to their potential therapeutic efficacy, researchers typically consider incorporating additional signaling motifs when designing CAR structures to further amplify CAR-T cell activation signals and prolong their *in vivo* half-life.

However, CAR-T cell therapy in the prior art also has certain limitations, such as antigen escape, on-target off-tumor effects (i.e., off-target effects), migration and tumor infiltration of CAR-T cells, and immunosuppressive microenvironments. In contrast to hematological malignancies, CAR-T cell therapy for solid tumors is limited by the ability of CAR-T cells to migrate into and infiltrate solid tumors, as the immunosuppressive tumor microenvironment and physical tumor barriers, such as tumor stroma, restrict the penetration and migration of CAR-T cells. In the tumor microenvironment, numerous cell types that drive immunosuppression can infiltrate solid tumors, including myeloid-derived suppressor cells (MDSCs), tumor-associated macrophages (TAMs), and regulatory T cells (Tregs). These tumor-infiltrating cells drive the production of tumor-promoting cytokines, chemokines, and growth factors. In addition, immune checkpoint pathways, such as PD-1 or CTLA-4, can be used to reduce anti-tumor immunity. One of the main reasons for the lack of response or weak response to CAR-T cell therapy is poor T cell expansion and short-term persistence of T cells.

Exosomes (Exo) are small vesicles with a lipid bilayer structure and a particle size of 30 to 150 nm. First, the cell membrane invaginates to generate early endosomes, which subsequently develop into late endosomes or multivesicular bodies (MVBs). Ultimately, the MVBs fuse with the cell membrane to release exosomes. The formation process of exosomes is closely related to molecules such as lipids and proteins on the cell membrane. The exosome membrane expresses relevant proteins derived from the cell membrane, as well as some marker proteins, such as tetraspanins (CD63, CD9, CD81, and CD82), heat shock protein 70 (HSP70), and tumor susceptibility gene 101 (TSG101) protein. Almost all cells can secrete exosomes, wherein the membrane proteins, contents, and functions of these exosomes may exhibit distinct characteristics. For example, exosomes derived from mesenchymal stem cells can inhibit tumor formation, growth, and invasion through the miRNAs therein, while exosomes derived from tumor cells can promote tumor migration by creating a microenvironment for the tumor or assist the tumor in immune evasion. Exosomes with different targeting categories and capabilities can be customized by processing exosome-derived cells through genetic engineering techniques or directly modifying exosomes, which gives exosomes broad prospects in the field of targeted tumor therapy.

Exosomes exhibit characteristics such as high biocompatibility, low immunogenicity, and ease of modification. Research related to the use of exosomes as carriers for drug delivery has been rapidly developed. Targeting determines whether exosomes can precisely and efficiently deliver drugs to diseased cells or tissues. Therefore, in order to improve the specificity of exosomes for tumor cells, exosomes with certain inherent targeting properties can be selected as drug delivery carriers, or exosomes can be modified by other means to enhance their targeting function, thereby further improving the clinical application potential and value of exosomes.

## SUMMARY

The present disclosure addresses the technical problem in the prior art where the therapeutic effectiveness and therapeutic safety of CAR-T cells targeting CD19 or CD22 are not high enough in clinical treatment. By employing gene editing technology, the variable region of T cells is replaced with a single-chain antibody, enabling the expression of chimeric antigen receptors (CARs) on the surface of T cells. The single-chain variable fragment (scFv) or ligand in CARs provides T cells with antibody-like specificity, allowing them to bind to target cells expressing corresponding antigens. The resulting T cells are referred to as chimeric antigen receptor T cells (CAR-T cells), which are utilized for tumor therapy.

In addition, the present disclosure addresses the problem in the prior art where the stroma of solid tumors impedes the penetration of CAR-T cells, making it difficult for CAR-T cells to infiltrate solid tumors, while the immunosuppressive tumor microenvironment also suppresses the immune response generated by CAR-T cell therapy, and clinical applications are often accompanied by toxic effects such as cytokine release syndrome and CAR-T cell-associated encephalopathy syndrome, resulting in limited efficacy of CAR-T cell therapy for solid tumors. Based on the characteristic that tumor cells actively uptake exosomes, exosomes containing CAR molecules are utilized for targeted tumor therapy.

To address the above technical problem, the present disclosure provides a technical solution as follows: a CAR molecule, wherein the CAR molecule is a CAR molecule targeting CD19 and/or a CAR molecule targeting CD22; the amino acid sequence of the CAR molecule targeting CD19 is as shown in SEQ ID NO: 1, and the amino acid sequence of the CAR molecule targeting CD22 is as shown in SEQ ID NO: 2.

In the present disclosure, the CAR molecule (chimeric antigen receptor) is an engineered transmembrane protein that combines the specificity of an antigen-specific antibody with the functionality of a T cell receptor. Generally, the CAR molecule is formed by the fusion of an extracellular antigen-targeting binding domain, a transmembrane domain, and an intracellular T cell activation signaling domain.

To address the above technical problem, the present disclosure provides a technical solution as follows: an isolated nucleic acid, wherein the nucleic acid encodes the CAR molecule according to the present disclosure.

In a preferred embodiment of the present disclosure, the nucleotide sequence encoding the CAR molecule targeting CD19 is as shown in SEQ ID NO: 3, and/or the nucleotide sequence encoding the CAR molecule targeting CD22 is as shown in SEQ ID NO: 4.

As is known in the art, "nucleic acid" in the present disclosure refers to a chain of nucleotides of any length, and includes DNA and RNA. The nucleotide may be a deoxyribonucleotide, a ribonucleotide, a modified nucleotide or base, and/or analogs thereof, or any substrate capable of being incorporated into a strand by a DNA or RNA polymerase.

To address the above technical problem, the present disclosure provides a technical solution as follows: a recombinant expression vector, wherein the recombinant expression vector comprises the nucleic acid according to the present disclosure.

In a preferred embodiment of the present disclosure, the recombinant expression vector is a lentiviral vector.

In a more preferred embodiment of the present disclosure, the backbone of the lentiviral vector is pLVX-EF1$\alpha$-IRES-Puro.

The term "recombinant expression vector" means a genetically modified oligonucleotide or polynucleotide construct that permits expression of an mRNA, protein, or peptide by a host cell, when the construct comprises a nucleotide sequence encoding the mRNA, protein, or peptide, and the vector is contacted with a cell under conditions sufficient to enable expression of the mRNA, protein, or peptide within the cell. The vectors of the present disclosure are generally not naturally occurring. However, portions of the vector may be naturally occurring. The recombinant expression vector of the present disclosure may comprise any type of nucleotides, including, but not limited to, DNA and RNA, which may be single-stranded or double-stranded, synthetic or partially derived from natural sources, and which may contain natural, non-natural, or altered nucleotides. The recombinant expression vector may comprise naturally occurring or non-naturally occurring internucleotide linkages, or both types of linkages. In exemplary aspects, the altered nucleotide or non-naturally occurring internucleotide linkage does not hinder transcription or replication of the vector.

The recombinant expression vector of the present disclosure may be any suitable recombinant expression vector, which can be used to transform or transfect one or more genes or sequences of interest into any suitable host cell and preferably express the genes or sequences in the host cell. Suitable vectors include those designed for expansion and amplification or for expression or both. Examples of vectors include, but are not limited to, viral vectors, naked DNA or RNA expression vectors, plasmids, cosmids or phage vectors, DNA or RNA expression vectors associated with cationic condensing agents, DNA or RNA expression vectors encapsulated in liposomes, and certain eukaryotic cells, such as producer cells.

To address the above technical problem, the present disclosure provides a technical solution as follows: a transformant, wherein the transformant expresses the CAR molecule according to the present disclosure.

In a preferred embodiment of the present disclosure, the transformant is a host cell transfected with the recombinant expression vector according to the present disclosure.

In a more preferred embodiment of the present disclosure, the host cell is a mammalian cell, such as a T cell, a 293 cell, or a cell line derived therefrom, such as a 293T cell or a 293F cell.

As used herein, the term "host cell" refers to any type of cell that may contain the nucleic acid or vector described herein. In exemplary aspects, the host cell is derived from or obtained from a mammal. A particularly preferred mammal is a human. In the present disclosure, the term "host cell" may include cells into which exogenous nucleic acids have been introduced, including progeny of these cells. Host cells include "transformants" or "transformed cells", which include primary transformed cells and progeny derived therefrom, regardless of the number of passages. The progeny may not be completely identical in nucleic acid content to a parent cell, but may contain mutations. Mutant progeny having the same function or biological activity as the cells screened or selected in the initially transformed cells are included herein.

For the purposes herein, the T cell may be any T cell, such as a cultured T cell, *e.g.*, a primary T cell, or a T cell derived from a cultured T cell line, e.g., Jurkat or SupT1, or a T cell obtained from a mammal. If obtained from a mammal, the T cell may be obtained from numerous sources including, but not limited to, blood, bone marrow, lymph nodes, thymus, or other tissues or fluids. The T cell may also be enriched or purified. The T cell may be obtained by maturation of hematopoietic stem cells into T cells *in vitro* or *in vivo.* In exemplary aspects, the T cell is a human T cell. In exemplary aspects, the T cell is a T cell isolated from a human. The T cell may be any type of T cell; preferably, the T cell is a CD3-positive T cell.

In the present disclosure, the 293T cell is a cell line derived from 293 cells (human embryonic kidney cells) through genetic technology, which is transfected with the adenovirus E1A gene, capable of expressing the SV40 large T antigen, and contains the SV40 origin of replication and promoter region. The 293F cell is a wild-type 293 cell line capable of high protein expression under serum-free conditions and suspension growth in medium.

The transformant according to the present disclosure, wherein the transformant further expresses granzyme and/or perforin; the amino acid sequence of the granzyme is as shown in SEQ ID NO: 10, and the amino acid sequence of the perforin is as shown in SEQ ID NO: 8.

In a preferred embodiment of the present disclosure, the granzyme and/or perforin is fused to CD63 (LAMP-3, lysosome-associated membrane protein-3), wherein the amino acid sequence of the CD63 is as shown in SEQ ID NO: 6.

In the present disclosure, the fusion refers to the formation of a fusion protein upon expression by connecting the nucleotide sequences of granzyme and/or perforin and CD63 using a conventional linker in the art.

In a more preferred embodiment of the present disclosure, the transformant further overexpresses an exosome secretion-promoting protein, wherein the exosome secretion-promoting protein is CX43, KIBRA, or Rab27a; the NCBI reference sequence number for the amino acid sequence of the CX43 is NP_000156.1, preferably with S replaced by A at position 368; the NCBI reference sequence number for the amino acid sequence of the KIBRA is NP_056053.1; the NCBI reference sequence number for the amino acid sequence of the Rab27a is NP_899058.1.

In a further preferred embodiment of the present disclosure, the nucleotide sequence encoding the granzyme is as shown in SEQ ID NO: 9; the nucleotide sequence encoding the perforin is as shown in SEQ ID NO: 7; the nucleotide sequence encoding the CD63 is as shown in SEQ ID NO: 5; the NCBI reference sequence number for the nucleotide sequence encoding the CX43 is NM_000165.5, preferably with AGC replaced by GCC at positions 1317 to 1319; the NCBI reference sequence number for the nucleotide sequence encoding the KIBRA is XM_005265853.3; and/or the NCBI reference sequence number for the nucleotide sequence encoding the Rab27a is NM_183236.3.

In the present disclosure, the "NCBI reference sequence number" refers to the nucleotide or amino acid sequence of a gene obtained by searching the biological database National Center for Biotechnology Information (https://www.ncbi.nlm.nih.gov/) using the NCBI Reference Sequence.

The transformant according to the present disclosure, wherein the genes encoding the granzyme, perforin, CD63, KIBRA, and/or Rab27a are located in an expression plasmid.

In a preferred embodiment of the present disclosure, the backbone of the expression plasmid is pcDNA3.1 or pCMV. The pcDNA3.1 or pCMV according to the present disclosure are plasmid vectors well known in the art.

To address the above technical problem, the present disclosure provides a technical solution as follows: a method for preparing an exosome, wherein the method comprises culturing the transformant according to the present disclosure, followed by isolation and purification to obtain the exosome from the culture medium of the transformant. The method for culturing the transformant is conventional in the art.

To address the above technical problem, the present disclosure provides a technical solution as follows: an exosome, wherein the exosome comprises the CAR molecule according to the present disclosure.

In a preferred embodiment of the present disclosure, the exosome is prepared by the method for preparing an exosome according to the present disclosure.

To address the above technical problem, the present disclosure provides a technical solution as follows: a pharmaceutical composition, wherein the pharmaceutical composition comprises one or more components selected from the group consisting of: the CAR molecule according to the present disclosure, the transformant according to the present disclosure, and the exosome according to the present disclosure.

In a preferred embodiment of the present disclosure, the pharmaceutical composition further comprises a pharmaceutically acceptable carrier.

The pharmaceutical composition of the present disclosure comprises a suitable pharmaceutically acceptable carrier, *e.g.*, a pharmaceutical excipient, such as a pharmaceutical carrier or a pharmaceutical vehicle known in the art, including a buffer. As used in the present disclosure, "pharmaceutically acceptable carrier" or "pharmaceutical carrier" includes any and all solvents, dispersion media, isotonic agents, absorption delaying agents, and the like that are physiologically compatible.

To address the above technical problem, the present disclosure provides a technical solution as follows: use of the CAR molecule according to the present disclosure, the transformant according to the present disclosure, the exosome according to the present disclosure, or the pharmaceutical composition according to the present disclosure in the manufacture of a medicament for preventing and/or treating a tumor associated with CD19 and/or CD22 expression.

In a preferred embodiment of the present disclosure, the tumor is acute B lymphoblastic leukemia.

In a more preferred embodiment of the present disclosure, the acute B lymphoblastic leukemia is B-ALL leukemia or aggressive B-cell lymphoma.

To address the above technical problem, the present disclosure provides a technical solution as follows: a method for diagnosing, treating, and/or preventing a tumor associated with CD19 and/or CD22 expression, wherein the method comprises administering to a patient in need thereof a therapeutically effective amount of the CAR molecule according to the present disclosure, the transformant according to the present disclosure, the exosome according to the present disclosure, or the pharmaceutical composition according to the present disclosure.

In a preferred embodiment of the present disclosure, the tumor is acute B lymphoblastic leukemia.

In a more preferred embodiment of the present disclosure, the acute B lymphoblastic leukemia is B-ALL leukemia or aggressive B-cell lymphoma.

As used in the present disclosure, the term "effective amount" refers to an amount of a drug or agent that elicits a biological or medical response in, for example, a tissue, system, animal, or human that is being sought by a researcher or clinician. In addition, the term "therapeutically effective amount" refers to an amount that results in improved treatment, cure, prevention, or alleviation of a disease, disorder, or side effect, or reduces the rate of progression of a disease or condition, as compared to a corresponding subject not receiving the amount. The term also includes within its scope an amount effective to enhance normal physiological function.

To address the above technical problem, the present disclosure provides a technical solution as follows: a combination therapy comprising administering to a patient in need thereof the CAR molecule according to the present disclosure, the transformant according to the present disclosure, the exosome according to the present disclosure, or the pharmaceutical composition according to the present disclosure, and a second therapeutic agent.

In a preferred embodiment of the present disclosure, the second therapeutic agent comprises other anti-tumor antibodies or a pharmaceutical composition comprising the other anti-tumor antibodies, and/or other anti-tumor drugs.

To address the above technical problem, the present disclosure provides a technical solution as follows: the CAR molecule according to the present disclosure, the transformant according to the present disclosure, the exosome according to the present disclosure, or the pharmaceutical composition according to the present disclosure for use in diagnosing, preventing, and/or treating a tumor associated with CD19 and/or CD22 expression.

In a preferred embodiment of the present disclosure, the tumor is acute B lymphoblastic leukemia.

In a more preferred embodiment of the present disclosure, the acute B lymphoblastic leukemia is B-ALL leukemia or aggressive B-cell lymphoma.

On the basis of common knowledge in the art, the above preferred conditions can be arbitrarily combined to obtain preferred examples of the present disclosure.

The reagents and raw materials used in the present disclosure are commercially available.

The positive and progressive effects of the present disclosure are as follows:

The CAR-T cells CAR19T and CAR22T prepared in the present disclosure exhibit higher therapeutic effectiveness and therapeutic safety in clinical treatment compared to similar CAR-T cells in the prior art, as evidenced by a higher complete remission rate, a higher overall response rate, along with lower incidence rates of severe CRS and neurotoxicity in patients.

Compared to CAR-T cells (CAR19T) incorporating the scFv of CD19 monoclonal antibody or CAR-T cells (CAR22T) incorporating the scFv of CD22 monoclonal antibody, the exosomes Exo-CAR-CD19 or Exo-CAR-CD22 produced by 293F cells can not only effectively lyse CD19-positive and CD22-positive Raji-luc tumor cells, but also reduce the adverse reactions caused by off-target CAR-T cells, indicating that the exosomes Exo-CAR-CD19 or Exo-CAR-CD22 exhibit better targeting performance and can achieve better tumor penetration. Therefore, Exo-CAR exosomes are expected to become a new direction of CAR-T cell therapy for the treatment of tumors.

BRIEF DESCRIPTION OF THE DRAWINGS

FIG. 1 shows a schematic diagram of the partial structures of CAR19 and CAR22.

FIG. 2 shows a schematic diagram of the structure of the recombinant lentiviral vector pCAR19.

FIG. 3 shows a schematic diagram of the structure of the recombinant lentiviral vector pCAR22.

FIG. 4 shows the results of the concentration assay of plasmid pCAR19.

FIG. 5 shows the results of the concentration assay of plasmid pCAR22.

FIG. 6 shows the results of the restriction enzyme digestion assay of plasmid pCAR19 and plasmid pCAR22.

FIG. 7 shows the results of the transduction efficiency assay after transduction of CAR19T cells and CAR22T cells.

FIG. 8 shows the results of the tumor-killing assay of CAR19T cells and CAR22T cells.

FIG. 9 shows the experimental results of the effects of CAR19T and CAR22T on tumor growth in mice.

FIG. 10 shows the results of secretion promotion of exosome secretion-promoting genes.

FIG. 11 shows the electron microscopy, particle size, and molecular phenotyping by flow cytometry of Exo-CAR-CD19 and Exo-CAR-CD22.

FIG. 12 shows the *in vivo* anti-tumor efficacy of Exo-CAR-CD19 and Exo-CAR-CD22.

FIG. 13 shows the determination of granzyme and perforin levels in engineered exosomes Exo-CAR-CD19 and Exo-CAR-CD22; wherein part A represents the determination of perforin levels, and part B represents the determination of granzyme levels.

FIG. 14 shows the tumor-killing efficiency of exosomes Exo-CAR-CD19 and Exo-CAR-CD22; wherein part A represents the killing efficiency of Exo-CAR-CD22, and part B represents the killing efficiency of Exo-CAR-CD19.

FIG. 15 shows the concentration gradient-mediated killing results of exosomes Exo-CAR-CD19 and Exo-CAR-CD22.

FIG. 16 shows the standard curve of perforin or granzyme.

FIG. 17 shows the total protein of the exosome secretion-promoting gene CX43 (S368A); wherein -CX43 indicates no transfection with the exosome secretion-promoting gene CX43 (S368A); +CX43 indicates transfection with the exosome secretion-promoting gene CX43 (S368A).

DETAILED DESCRIPTION OF THE PREFERRED EMBODIMENT

The present disclosure is further described below by way of examples, but the present disclosure is not limited to the scope of the described examples. Experimental methods for which specific conditions are not indicated in the following examples are selected in accordance with conventional methods and conditions, or in accordance with the product instructions.

Sources of the biological and reagent materials in the present disclosure:

pLVX-EF1α-IRES-Puro: purchased from Clontech.
DH5alpha competent cells: purchased from Takara.
EndoFree Plasmid Mega Kit: purchased from Qiagen, including QIAfilter Cartridge, Buffers P1, Buffers P2, Buffers P3, Buffer FW, Buffer ER, Buffers QBT, Buffer QC, Buffer QN, Endotoxin-free water, and Buffer TE.
gag plasmid and vsvg plasmid: purchased from Addgene.
293T cells: purchased from Takara.

Definitions of abbreviations and key terms in the present disclosure:

CAR-T: chimeric antigen receptor modified T cells
Exo-CAR: Exosome-chimeric antigen receptor; exosomes carrying CAR molecules

**Example 1: Construction of recombinant lentiviral vectors pCAR19 and pCAR22**

The amino acid sequence of CAR19 is as shown in SEQ ID NO: 1:

MDFQVQIFSFLLISASVIMSRMADIQMTQTTSSLSASLGDRVTISCRASQDISK

YLNWYQQKPDGTVKLLIYHTSRLHSGVPSRFSGSGSGTDYSLTISNLEQEDIATYFCQ

QGNTLPYTFGGGTKLEITGSTSGSGKPGSGEGSTKGEVKLQESGPGLVAPSQSLSVTC

TVSGVSLPDYGVSWIRQPPRKGLEWLGVIWGSETTYYNSALKSRLTIIKDNSKSQVFL

KMNSLQTDDTAIYYCAKHYYYGGSYAMDYWGQGTSVTV

The amino acid sequence of CAR22 is as shown in SEQ ID NO: 2:

MALPVTALLLPLALLLHAAIPDTEVQLVESGGGLVKPGGSLKLSCAASGFAFS

IYDMSWVRQTPEKRLEWVAYISSGGGTYYPDTVKGRFTISRDNAKNTLYLQMSSLKS

EDTAMYYCARHSGYGTHWGVLFAYWGQGTLVTVSAGGGGSGGGGSGGGGSDIQM

TQTTSSLSASLGDRVTISCRASQDISNYLNWYQQKPDGTVKLLIYYTSILHSGVPSRFS

GSGSGTDYSLTISNLEQEDFATYFCQQGNTLPWTFGGGTKLEIKATTTPAPRPPTPAPTI

ASQPLSLRPEACRPAAGGAVHTRGLDFACDFWVLVVVGGVLACYSLLVTVAFIIFWV

RSKRSRLLHSDYMNMTPRRPGPTRKHYQPYAPPRDFAAYRSKRGRKKLLYIFKQPFM

RPVQTTQEEDGCSCRFPEEEEGGCELRVKFSRSADAPAYQQGQNQLYNELNLGRREE

YDVLDKRRGRDPEMGGKPRRKNPQEGLYNELQKDKMAEAYSEIGMKGERRRGKGH

DGLYQGLSTATKDTYDALHMQALPPR

According to Sequence 1 (CAR19, SEQ ID NO: 3) and Sequence 2 (CAR22, SEQ ID NO: 4), the genes were synthesized by Nanjing GenScript Biotech Co., Ltd., and the synthesized sequences were cloned into a T vector.

Nucleotide sequence of CAR19 (SEQ ID NO: 3):

ATGGATTTTCAGGTGCAGATTTTCAGCTTCCTGCTAATCAGTGCCTCAGTC
ATAATGTCTAGAATGGCCGACATCCAGATGACCCAGACCACCTCCAGCCTGAGCG
CCAGCCTGGGCGACCGGGTGACCATCAGCTGCCGGGCCAGCCAGGACATCAGCA
AGTACCTGAACTGGTATCAGCAGAAGCCCGACGGCACCGTCAAGCTGCTGATCTA
CCACACCAGCCGGCTGCACAGCGGCGTGCCCAGCCGGTTTAGCGGCAGCGGCTC
CGGCACCGACTACAGCCTGACCATCTCCAACCTGGAACAGGAAGATATCGCCACC
TACTTTTGCCAGCAGGGCAACACACTGCCCTACACCTTTGGCGGCGGAACAAAGC
TGGAAATCACCGGCAGCACCTCCGGCAGCGGCAAGCCTGGCAGCGGCGAGGGCA
GCACCAAGGGCGAGGTGAAGCTGCAGGAAAGCGGCCCTGGCCTGGTGGCCCCCA
GCCAGAGCCTGAGCGTGACCTGCACCGTGAGCGGCGTGAGCCTGCCCGACTACG
GCGTGAGCTGGATCCGGCAGCCCCCCAGGAAGGGCCTGGAATGGCTGGGCGTGA
TCTGGGGCAGCGAGACCACCTACTACAACAGCGCCCTGAAGAGCCGGCTGACCA
TCATCAAGGACAACAGCAAGAGCCAGGTGTTCCTGAAGATGAACAGCCTGCAGA
CCGACGACACCGCCATCTACTACTGCGCCAAGCACTACTACTACGGCGGCAGCTA
CGCCATGGACTACTGGGGCCAGGGCACCAGCGTGACCGTG

Nucleotide sequence of CAR22 (SEQ ID NO: 4):

ATGGCCTTACCAGTGACCGCCTTGCTCCTGCCGCTGGCCTTGCTGCTCCAC
GCCGCCATTCCCGACACTGAAGTCCAGCTCGTGGAATCTGGAGGGGGCCTGGTGA
AACCTGGGGGATCTCTCAAACTGTCTTGTGCCGCTTCTGGCTTTGCTTTTAGCATC

TACGACATGTCCTGGGTCCGGCAGACACCTGAAAAACGCCTGGAGTGGGTCGCCT

ACATTTCTAGTGGGGGCGGAACATACTACCCCGATACCGTGAAGGGACGCTTTACA

ATTTCTAGGGATAACGCCAAAAACACCCTGTACCTCCAGATGTCATCCCTGAAATC

TGAGGATACTGCCATGTACTACTGTGCTAGGCATTCTGGCTACGGAACACATTGGG

GAGTGCTCTTCGCTTACTGGGGCCAGGGGACTCTCGTCACTGTCTCTGCTGGCGG

GGGAGGCTCTGGCGGAGGCGGATCCGGAGGCGGAGGGAGTGATATTCAGATGAC

TCAGACCACCTCTTCTCTGTCCGCTTCTCTGGGCGATAGAGTGACAATCTCCTGTC

GGGCATCACAGGATATTAGCAATTACCTGAACTGGTACCAGCAGAAACCCGATGG

AACCGTCAAACTGCTCATCTACTACACCTCCATCCTCCACTCTGGCGTGCCATCTC

GATTTCTGGATCTGGCTCTGGAACCGACTACTCTCACAATCTCCAACCTGGAA

CAGGAGGATTTTGCCACCTACTTTTGTCAGCAGGGCAATACTCTGCCTTGGACCTT

TGGGGGCGGAACCAAACTGGAAATCAAGGCCACCACGACGCCAGCGCCGCGACC

ACCAACACCGGCGCCCACCATCGCGTCGCAGCCCCTGTCCCTGCGCCCAGAGGCG

TGCCGGCCAGCGGCGGGGGGCGCAGTGCACACGAGGGGGCTGGACTTCGCCTGT

GATTTTGGGTGCTGGTGGTGGTTGGTGGAGTCCTGGCTTGCTATAGCTTGCTAGT

AACAGTGGCCTTTATTATTTTCTGGGTGAGGAGTAAGAGGAGCAGGCTCCTGCAC

AGTGACTACATGAACATGACTCCCCGCCGCCCCGGGCCCACCCGCAAGCATTACC

AGCCCTATGCCCCACCACGCGACTTCGCAGCCTATCGCTCCAAACGGGGCAGAAA

GAAACTCCTGTATATATTCAAACAACCATTTATGAGACCAGTACAAACTACTCAAG

AGGAAGATGGCTGTAGCTGCCGATTTCCAGAAGAAGAAGAAGGAGGATGTGAAC

TGAGAGTGAAGTTCAGCAGGAGCGCAGACGCCCCCGCGTACCAGCAGGGCCAGA

ACCAGCTCTATAACGAGCTCAATCTAGGACGAAGAGAGGAGTACGATGTTTTGGA

CAAGAGACGTGGCCGGGACCCTGAGATGGGGGGAAAGCCGAGAAGGAAGAACC

CTCAGGAAGGCCTGTACAATGAACTGCAGAAAGATAAGATGGCGGAGGCCTACA

GTGAGATTGGGATGAAAGGCGAGCGCCGGAGGGGCAAGGGGCACGATGGCCTTT

ACCAGGGTCTCAGTACAGCCACCAAGGACACCTACGACGCCCTTCACATGCAGGC

CCTGCCCCCTCGCTAA

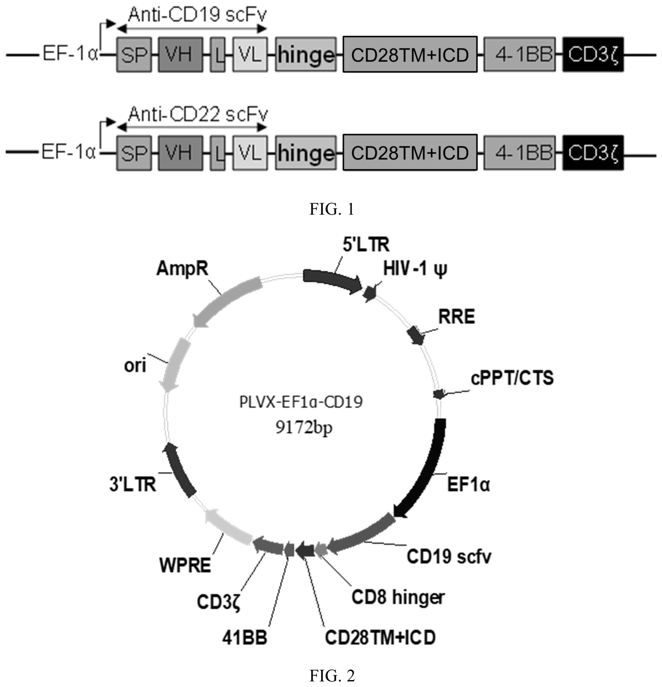

The partial structures of CAR19 and CAR22 are shown in FIG. 1, wherein both CD19 and CD22 are third-generation CARs comprising CD28 and 4-1BB as co-stimulatory signals. The sequencing comparison results of CAR19 and CAR22 showed complete sequence matching.

1. The CAR19 sequence fragment with a length of 798 bp was designed with EcoRI and MluI restriction sites at both ends and cloned into the multiple cloning site of the lentiviral backbone plasmid pLVX-EF1α-IRES-Puro to complete the vector construction. The complete construction map is shown in FIG. 2, wherein WPRE represents woodchuck hepatitis virus posttranscriptional regulatory element, RRE represents Rev response element, and cPPT/CTS represents central polypurine tract/central termination sequence.

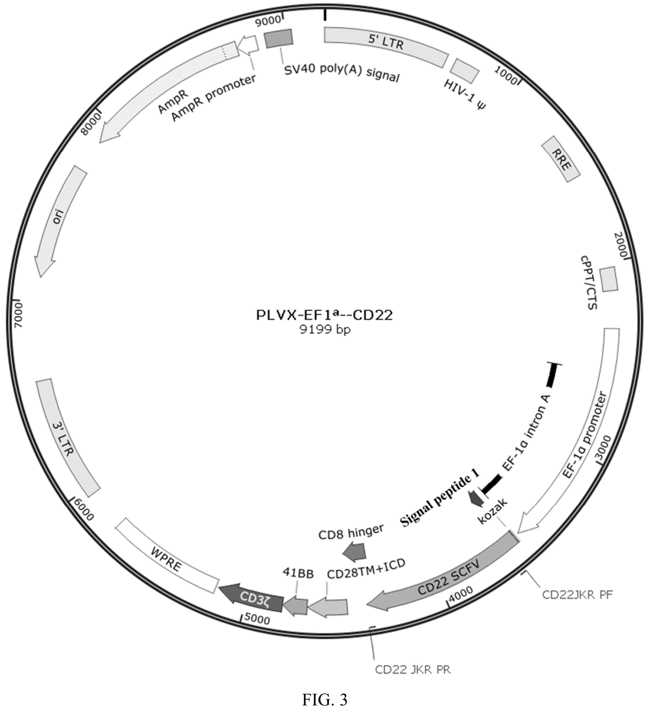

2. The CAR22 sequence fragment with a length of 1608 bp was designed with EcoRI and MluI restriction sites at both ends and cloned into the multiple cloning site of the lentiviral backbone plasmid pLVX-EF1α-IRES-Puro to complete the vector construction. The complete construction map is shown in FIG. 3.

The lentiviral backbone plasmid pLVX-EF1α-IRES-Puro and the synthetic sequence were double-digested using EcoRI-HF and MluI restriction enzymes. The products were subjected to 1.5% agarose gel electrophoresis, and the gel slices were excised and recovered in an Eppendorf tube. The corresponding fragments were recovered using the Agarose Gel Extraction Kit from QIAGEN, and the purity and concentration of the products were measured.

The fragments were added to the Eppendorf tube at a molar ratio of 1:1, followed by the addition of T4 DNA ligase (NEB) and T4 DNA ligase buffer. The reaction was carried out at 22°C for 2 hours. Subsequently, 8 μL of the ligation mixture was transferred to 100 μL of DH5alpha competent cells, incubated in an ice bath for 30 minutes, and heat-shocked at 42°C for 90 seconds. After completion, 500 μL of SOC medium was added, and the mixture was incubated at 37°C and 220 rpm for 2 hours. After 2 hours, the Eppendorf tube was centrifuged at 4000 g for 1 minute to remove 400 μL of excess liquid. The remaining liquid was spread on an LB plate and incubated at 37°C for 12 hours. Single colonies were picked from the plate and inoculated into 5 mL of LB liquid medium, followed by incubation at 37°C and 220 rpm for 12 hours.

The plasmids pCAR19 and pCAR22 were extracted using the Miniprep Kit from QIAGEN. After verification by Sanger sequencing at Nanjing GenScript Biotech Co., Ltd., DH5alpha strains containing pCAR19 or pCAR22 were preserved.

**Example 2: Preparation of plasmids pCAR19 and pCAR22**

DH5alpha strains containing pCAR19 or pCAR22 were inoculated into 250 mL of LB medium containing 100 μg/mL ampicillin and incubated overnight at 37°C and 220 rpm. The medium was centrifuged at 4°C and 6000 g for 20 minutes, and the supernatant was discarded.

Buffers P1 was removed from the EndoFree Plasmid Mega Kit (Qiagen), and 120 mL of pre-chilled Buffers P1 was added to the *E. coli* pellet obtained by centrifugation. The centrifuge bottle was capped and vigorously shaken to completely disperse the *E. coli* pellet in Buffers P1.

120 mL of Buffer P2 was added to the centrifuge bottle, which was capped and placed on a roller mixer. The speed was gradually increased to 50 rpm. After thorough mixing, the mixture was allowed to stand at room temperature for 5 minutes.

120 mL of Buffer P3 was added to the centrifuge bottle, which was capped and placed on a roller mixer. The speed was gradually increased to the maximum speed of the roller mixer at 70 rpm. After thorough mixing, a white, non-viscous, fluffy mixture was obtained. The mixture was centrifuged at 4°C and 9000 g for 15 minutes.

50 mL of Buffer FW was poured into the QIAfilter Cartridge, and the supernatant obtained by centrifugation was poured into the QIAfilter Cartridge, followed by gentle mixing. The mixture was filtered into a vial labeled correspondingly.

20 mL of Buffer ER was added to each vial, which was mixed by inverting 6 times and incubated at -20°C for 30 minutes.

The labeled Mega columns were placed on the corresponding racks. 35 mL of Buffers QBT was added to each Mega column for equilibration, and allowed to flow through by gravity.

All the liquid in the vial was poured into the corresponding labeled Mega columns in batches. After the liquid in the column had been drained, 200 mL of Buffer QC was added to each Mega column in batches for washing. After the liquid in the column had been drained, the waste liquid in the waste collection tray was poured into a clean 50 mL centrifuge tube.

40 mL of Buffer QN was added to each Mega column. The effluent was collected in a clean 50 mL centrifuge tube, which was mixed by inverting 6 times, and 20 mL of the mixture was aliquot into another clean labeled 50 mL centrifuge tube.

14 mL of isopropanol (room temperature) was added to each 50 mL centrifuge tube, which was mixed by inverting 6 times. The mixture was centrifuged at 4°C and 15000 g for 50 minutes.

The supernatant was completely aspirated in a clean bench, and 3.5 mL of Endotoxin-free water was added to each tube for rinsing, without disturbing the precipitate at the bottom. The mixture was centrifuged at 4°C and 15000 g for 30 minutes. Buffer TE from the EndoFree Plasmid Mega Kit was preheated in an oven.

The supernatant obtained by centrifugation was completely aspirated in the clean bench, followed by blow-drying (to evaporate residual absolute ethanol, with a duration of approximately 10 minutes) in the clean bench.

Buffer TE was taken out from the oven, and 1 mL of Buffer TE was added to each tube in the clean bench. The mixture was pipetted 10 times and then placed in an oven at 65°C, during which the tube wall was continuously tapped to facilitate complete dissolution of the precipitate. The tube was centrifuged at 4°C and 4000 g for 1 minute to pellet the liquid on the tube wall to the bottom of the tube, followed by mixing by pipetting.

All the liquid was transferred to endotoxin-free, pyrogen-free, and nuclease-free EP tubes labeled correspondingly in the clean bench. 2 μL of the mixture was aspirated, and the plasmid concentration was measured using a micro-spectrophotometer and labeled on the corresponding EP tube to obtain pCAR19 or pCAR22.

Plasmid assay:

1. Plasmid concentration assay

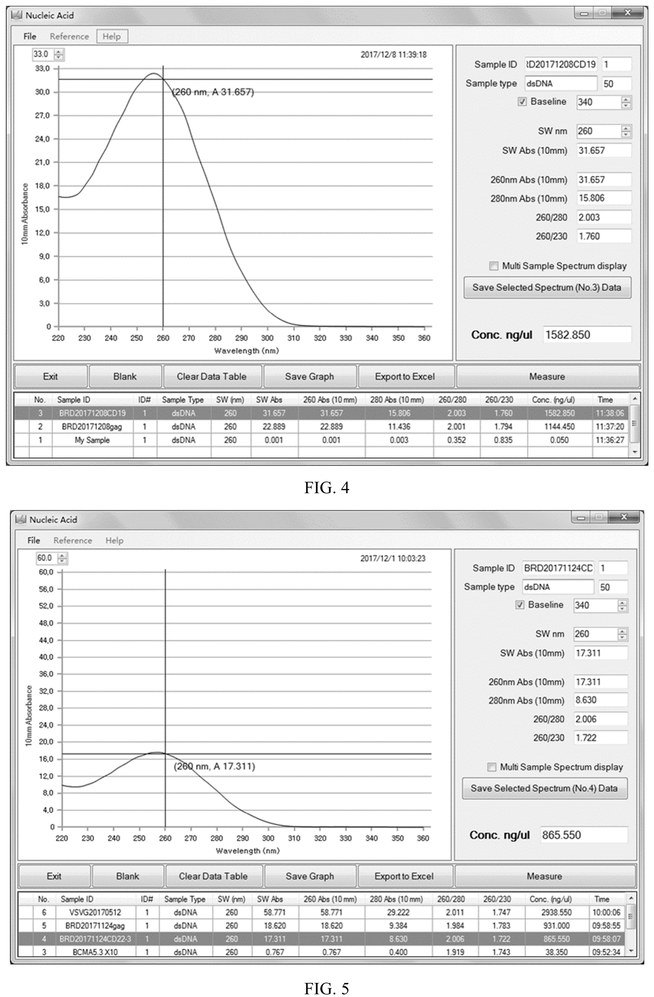

The sample was collected, and 1 μL was taken for concentration determination using an ultra-micro UV spectrophotometer (NanoDrop). The nucleic acid measurement module was selected, parameters were set, and assay was performed after blank correction. The results are shown in FIG. 4, FIG. 5, and Table 1 below:

Table 1

| Sample name | Sample properties | Sample volume (μL) | NanoDrop assay | | |
|---|---|---|---|---|---|
| | | | Conc. (ng/μL) | 260/280 | 260/230 |
| pCAR19 | Clear and transparent | 50 | 1582.85 | 2.003 | 1.76 |
| pCAR22 | Clear and transparent | 50 | 865.55 | 2.006 | 1.722 |

2. Plasmid DNA (restriction enzyme digestion) assay

Principle: Agarose gel electrophoresis is a standard method for separating, identifying, and purifying DNA fragments. Agarose is a polysaccharide extracted from agar, which is hydrophilic but uncharged and serves as an excellent electrophoretic support medium. DNA is negatively charged under alkaline conditions (in a pH 8.0 buffer) and migrates toward the positive electrode through a gel medium in an electric field. Different DNA molecular fragments exhibit varying electrophoretic mobility rates in the electric field due to differences in molecular size and conformation. Ethidium bromide (EB) can intercalate between the base pairs of DNA molecules to form a fluorescent complex, which, upon UV irradiation, can be separated into distinct bands, thereby achieving the purpose of separation, determination of molecular weight, and screening of recombinants.

The results of enzyme digestion can be used to preliminarily determine the supercoiled plasmid content in the stock solution. The higher the supercoiled content, the better the plasmid purity and the better the efficiency of subsequent viral packaging.

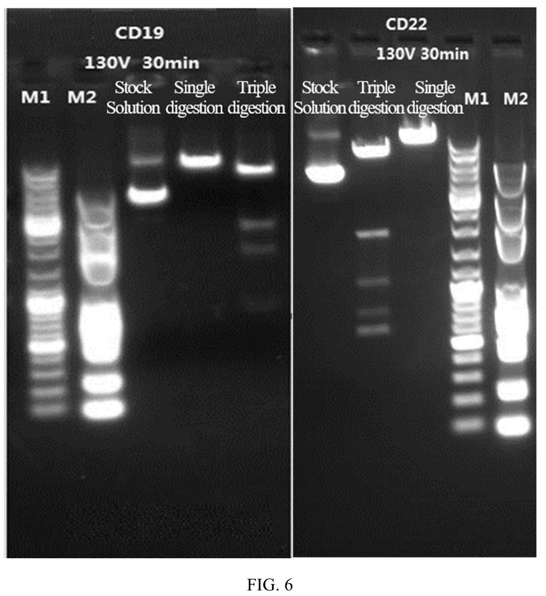

Method: 200 ng of each sample was taken, followed by triple digestion with EcoRI, KpnI, and MluI and single digestion with EcoRI, and analyzed by 0.7% agarose gel electrophoresis. Sample numbers were labeled above the gel wells: M1: 10000 kb DNA Marker (10000, 8000, 6000, 5000, 4000, 3500, 3000, 2000, 1500, 1200, 1000, 900, 800, 700, 600, 500, 400, 300, 200, 100); M2: DL5000 DNA Marker (5000, 3000, 2000, 1000, 750, 500, 250, 100). The results of agarose gel electrophoresis (approximately 100 ng of sample loaded) is shown in FIG. 6.

3. Target gene sequencing

20 μL (500 ng) of plasmid DNA was taken and sent for sequencing. Based on the original seed sequence, it was verified whether the target gene in the product obtained from plasmid production had undergone any changes. Under stable processing conditions, the working seed did not exhibit alterations in the target gene during the fermentation scale-up process and could be used for subsequent production and correct protein expression.

**Example 3: Preparation of recombinant lentiviruses LVCAR19 and LVCAR22**

130.0 to $140.0 \times 10^6$ 293T cells (Takara) were inoculated into a multi-layer cell culture flask (Hyperflask) (Corning) containing a total of 560 mL of DMEM complete medium (50 mL of fetal bovine serum, 5 mL of Antibiotic-Antimycotic ($100\times$)). The mixture was incubated in an incubator at 37°C with 5% $CO_2$ for 24 hours. DMEM complete medium mixed with 320 μg of plasmid (pCAR19 or pCAR22: gag plasmid: vsvg plasmid = 6:3:2) was added to a tube containing 960 μg of PEI, vortexed, and equilibrated at room temperature for 10 minutes. 35 mL of the PEI-plasmid mixture was mixed with 525 mL of DMEM complete medium and transferred into the multi-layer cell culture flask described above. The multi-layer cell culture flask was incubated in an incubator at 37°C with 5% $CO_2$ for 3 days, and the cell culture supernatant was collected.

The supernatant was centrifuged at 4000 rpm (or 3000 g) for 30 minutes, and Cryonase nuclease (Takara) was added to the centrifuged supernatant and incubated at 4°C. After 6 hours, the lentiviral supernatant was filtered through a 0.22 $\mu$m membrane and centrifuged at 4°C and 30,000 g for 2.5 hours. The supernatant was discarded, and the pellet was resuspended in 1 mL of T cell medium. After resuspension, 20 $\mu$L was retained for activity titer assay. The remaining lentivirus concentrate was aliquoted, labeled as LVCAR19 or LVCAR22, and stored at -80°C for later use.

Lentiviral activity titer assay:

Principle: Protein-L is labeled with fluorescein, and can specifically bind to the Kappa region of the light chain of a single-chain antibody in CAR. The fluorescence signal detected by flow cytometry indirectly reflects the expression of CAR in 293T cells.

Method: $5.0 \times 10^5$ 293T cells per well were inoculated into a 6-well plate, followed by the addition of 0.1 $\mu$L, 0.5 $\mu$L, and 1 $\mu$L of lentivirus concentrate to each well, respectively, and one negative control was set up. The plate was incubated in an incubator at 37°C with 5% $CO_2$. After three days, 293T cells were collected using Versene solution (Gibco) for flow cytometry to determine the proportion of CAR-positive 293T cells, and the active titer of the LVCAR19 or LVCAR22 concentrate was calculated.

The current activity titer of the lentivirus stock solution ranges from 1 to 10E+05, while the activity titer of the lentivirus concentrate ranges from 1 to 10E+08. The detection and analysis results are shown in Tables 2 and 3:

Table 2

| CD22 sample No. | Amount of virus added ($\mu$L) | Transfection efficiency | Activity titer |
|---|---|---|---|
| 01 | Control (CK) | 1.62% | / |
| 02 | 0.1 | 6.11% | 3.06E+08 |
| 03 | 0.5 | 26.6% | 2.67E+08 |
| 04 | 1 | 49.0% | 2.45E+08 |

Table 3

| CD19 sample No. | Amount of virus added ($\mu$L) | Transfection efficiency | Activity titer |
|---|---|---|---|
| 01 | Control (CK) | 0.55% | / |
| 02 | 0.1 | 11.20% | 5.60E+08 |
| 03 | 0.5 | 33.00% | 3.30E+08 |
| 04 | 1 | 79.30% | 3.95E+08 |

**Example 4: Preparation of CAR19T and CAR22T**

100 mL of peripheral blood was collected from a healthy donor, and mononuclear cells were isolated using Ficoll lymphocyte separation medium. After counting, CD3-positive cells were sorted using an appropriate amount of CD3 MicroBeads, human (Miltenyi Biotec) and cultured at a density of 1.0 to $2.0 \times 10^6$ cells/mL in T cell complete medium (OpTmizer™ CTS™ T-Cell Expansion Basal Medium, OpTmizer™ CTS T-Cell Expansion Supplement (Invitrogen), 500 U/mL of IL-2 (Beijing SL Pharmaceutical Co., Ltd.)), while Dynabeads Human T-Activator CD3/CD28 (Invitrogen) was added at 25 $\mu$L/$10^6$ cells to activate the T cells.

After 24 hours, LVCAR19 or LVCAR22 was added at an MOI of 3 for transduction, mixed well, and incubated in a $CO_2$ incubator for 4 hours, after which an appropriate amount of T cell complete medium was supplemented for culture.

24 hours after lentiviral transduction, the transduced CAR19T cells or CAR22T cells were transferred into fresh T cell complete medium, and the viable cell density was adjusted to $1.0 \times 10^6$ cells/mL. The cells were further cultured and expanded for 10 to 20 days, with daily observation and counting. Based on the number of cells counted, the culture was supplemented and scaled up, maintaining a constant cell culture density of $1.0 \times 10^6$ cells/mL.

Preparation of CAR-T cell formulation:

CAR19T cells or CAR22T cells were collected according to the estimated number of cells, resuspended in 100 mL of saline containing 2% human albumin, transferred into a cell infusion bag, and heat-sealed to prepare a finished CAR19T or CAR22T cell formulation.

CAR19T and CAR22T transduction efficiency assay:

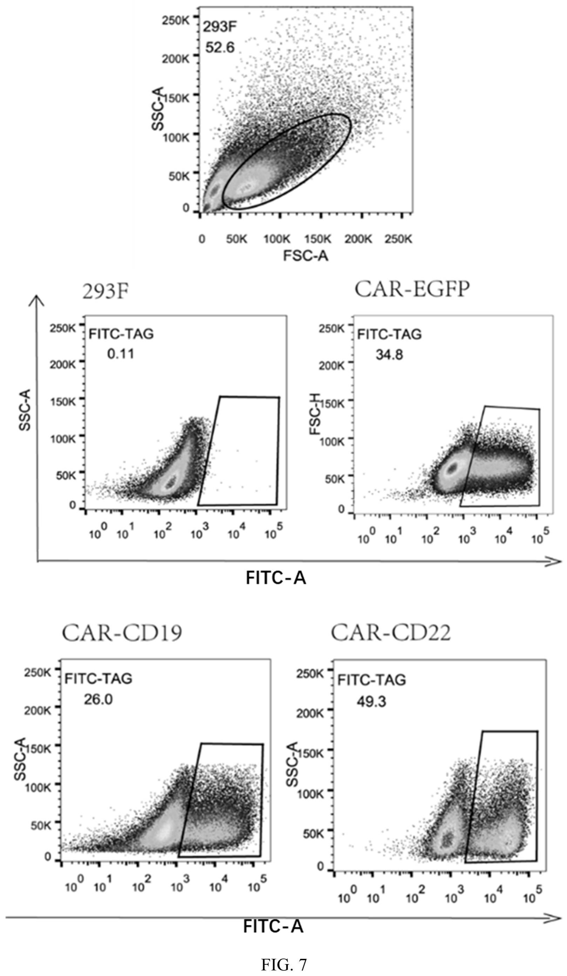

$1.0\times10^6$ transduced T cells were taken and incubated with 1 $\mu$g/mL FITC-Protein-L at room temperature for 30 minutes. After washing twice with saline, the FITC fluorescence signal was detected by flow cytometry, and the proportion of FITC-positive cells was measured, reflecting the proportion of CAR-T cells in the total cell population. The test results are shown in FIG. 7 and Table 4 below. It was demonstrated that CAR19T and CAR22T cells were successfully prepared.

Table 4

| No. | Transduction type | Transduction efficiency |
|---|---|---|
| 2017112007 | CAR19T | 51.0% |
| 2017112008 | CAR22T | 38.6% |

**Example 5: *In vitro* functional assay of CAR19T and CAR22T**

The *in vitro* tumor-killing function of CAR19T and CAR22T was evaluated using Calcein-AM.

An appropriate amount of K562 or RAJI target cells was taken, and Calcein-AM was added to a cell suspension (PBS, 5% fetal bovine serum) at $1\times10^6$ cells/mL to a final concentration of 25 $\mu$M, followed by incubation in an incubator for 30 minutes. The cells were washed twice at room temperature and resuspended to $1.5\times10^5$ cells/mL. CAR19T or CAR22T cells were added at different effector-to-target ratios, centrifuged at 200 g for 30 seconds, and incubated at 37°C for 2 to 3 hours. After incubation, the supernatant was collected, the fluorescence intensity of calcein was measured, and the percentage of target cell lysis was calculated based on the spontaneous release control and the maximum release control.

1. Tumor-killing assay data

Different batches of viruses require functional assays, such as tumor-killing assays in cell lines, prior to infusion. The calcein assay method was used with the formula as follows:

$$\text{Corrected \% lysis} = \frac{\text{F assay} - \text{F spontaneous}}{\text{F max} - \text{F spontaneous}}$$

F CTL assay = mean fluorescence value of three replicate wells containing target cells and effector T cells at a specific concentration

F spontaneous release = mean fluorescence value of three replicate wells containing target cells and $1\times$ PBS

F maximum release = mean fluorescence value of three replicate wells containing target cells and lysis buffer

Note: The fluorescence values of cell pellet lysates in the 96-well plate were measured simultaneously. The total fluorescence value for each well was equal to the sum of the fluorescence values of the supernatant and the cell pellet, with the difference in total fluorescence values controlled to less than 5%.

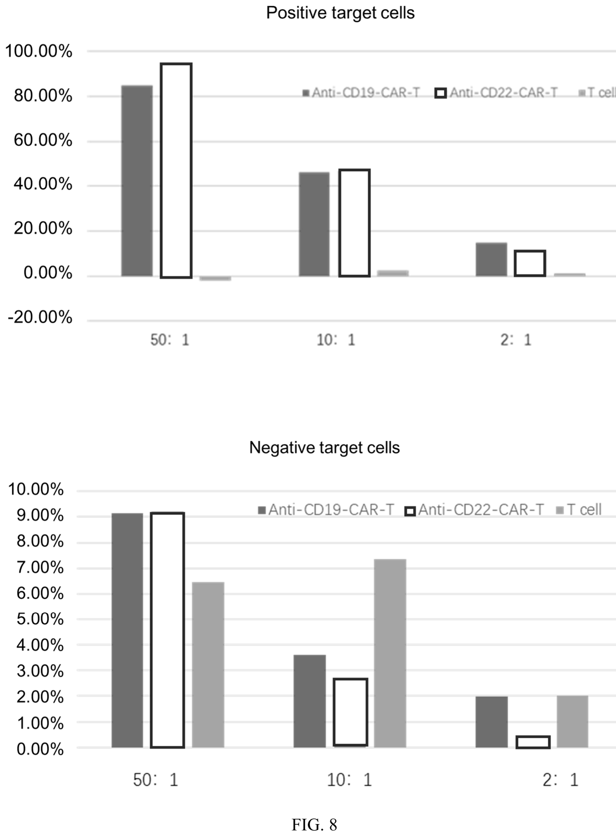

The results are shown in FIG. 8 and Tables 5 and 6 below. Anti-CD19-CAR-T is a CAR-T cell targeting CD19, and Anti-CD22-CAR-T is a CAR-T cell targeting CD22, with positive cells being RAJI cells and negative cells being K562 cells.

Table 5

| Positive target cells: | | | |
|---|---|---|---|
| Effector-to-target ratio | Anti-CD19-CAR-T | Anti-CD22-CAR-T | T cell |
| 50:1 | 84.84% | 94.40% | -2.12% |
| 10:1 | 46.26% | 46.92% | 2.32% |
| 2:1 | 14.86% | 11.46% | 1.28% |

Table 6

| Negative target cells: | | | |
|---|---|---|---|
| Effector-to-target ratio | Anti-CD19-CAR-T | Anti-CD22-CAR-T | T cell |
| 50:1 | 9.13% | 9.21% | 6.47% |
| 10:1 | 3.61% | 2.67% | 7.34% |
| 2:1 | 1.99% | 0.45% | 2.02% |

The results demonstrated that T cells loaded with Anti-CD19-CAR molecules exhibited killing rates of 85%, 46%, and 15% at effector-to-target ratios of 50:1, 10:1, and 2:1, respectively; while T cells loaded with Anti-CD22-CAR molecules exhibited killing rates of 94%, 47%, and 11% at effector-to-target ratios of 50:1, 10:1, and 2:1, respectively, indicating that the killing of tumor cells was highly efficient and dose-dependent.

**Example 6: Sequential infusion of CAR19T and CAR22T in a mouse tumor-bearing model**

To evaluate the anti-tumor efficacy of CAR-T cells *in vivo,* immunodeficient (NOD-SCID) mice and Raji-luc cells were selected to establish a tumor model. After successful modeling, CAR-T cells were injected, and the experimental results were detected using an IVIS small animal *in vivo* imaging system (Xenogen, Hopkinton, USA). The experimental steps were as follows:

1. Modeling: 5-week-old NOD-SCID mice were subcutaneously injected with Raji-luc cells at $5 \times 10^6$ cells/mouse.
2. After 7 days, the mice were imaged by intraperitoneal injection of D-luciferin (Molecular Imaging Products, Bend, USA) at a dose of 150 mg/kg, followed by anesthesia by intraperitoneal injection of sodium pentobarbital at a dose of 75 mg/kg. After 10 minutes, the optical signals were collected using the IVIS small animal *in vivo* imaging system (Xenogen, Hopkinton, USA).
3. On the day of successful modeling, CAR-T cells were injected, with the following groups:

(1) CD19 group, injected with CAR19T cells at $5 \times 10^6$ cells/mouse;
(2) CD22 group, injected with CAR22T cells at $5 \times 10^6$ cells/mouse;
(3) CD19+CD22 group, first injected with CAR19T cells at $5 \times 10^6$ cells/mouse, followed by injection with CAR22T cells at $5 \times 10^6$ cells/mouse 3 days later;
(4) NT group, injected with normal T cells at $5 \times 10^6$ cells/mouse;
(5) GFP group, injected with GFP lentivirus-transfected T cells at $5 \times 10^6$ cells/mouse.

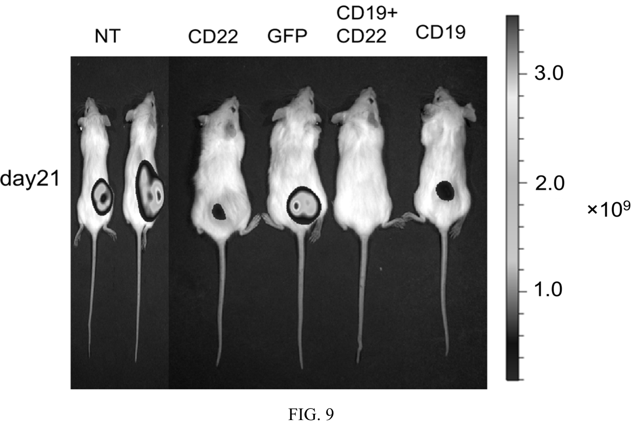

4. Imaging was performed 21 days after the injection of CAR-T cells, and the experimental results were analyzed, as shown in FIG. 9, wherein NT: normal T cell injection group; CD22: CAR22T injection group; GFP: GFP lentivirus-transfected T cell injection group; CD19+CD22: CAR19T and CAR22T injection group; CD19: CAR19T injection group.

The results demonstrated that compared to the normal T cell injection group and the GFP-T injection group, the CAR19T injection group and the CAR22T injection group exhibited significantly reduced tumor burden, while the CD19+CD22 injection group exhibited complete tumor eradication, indicating that the efficacy of sequential infusion of dual-target CAR-T cells was superior to that of single-target infusion and non-infusion groups.

**Example 7: Clinical application of CAR19T and CAR22T**

A total of 59 patients with relapsed/refractory/high-risk acute B lymphoblastic leukemia, including 42 patients with B-ALL and 17 patients with aggressive B-cell lymphoma, were treated with sequential infusion of anti-CD19 CAR-T and anti-CD22 CAR-T.

Clinical background of enrolled patients:

Regarding patients with B-ALL leukemia: the median age was 28 years, with a male-to-female ratio of approximately 3:2. Nearly half of the patients experienced more than two relapses or had primary refractory disease. Prior to participating in this trial, 7 patients underwent transplantation and 6 patients received CAR19 therapy, but all of them experienced relapse. The proportion of uncontrolled early B cells derived from the bone marrow ranged from 0 to 92%. Uncontrolled early B cells were identified in the cerebrospinal fluid of 6 patients. Most patients developed high-risk genetic aberrations, including BCR/ABL translocation, T315I mutation, 17p deletion, or TP53 mutation. MLL rearrangements, primarily MLL/AF4 and hypodiploidy, were also detected.

Regarding patients with aggressive B-cell lymphoma: the median age of lymphoma patients was 38 years, with a male-to-female ratio of approximately 1:1. All pathological subtypes were highly malignant B-cell lymphomas, including one case of double-hit lymphoma. Nearly 65% of the patients experienced more than two relapses or had primary refractory disease. 3 patients underwent transplantation but experienced relapse. Nearly half of the patients carried Myc translocation, 17p deletion, or TP53 mutation.

Effectiveness of CAR-T cell therapy:

Among patients with leukemia, 40 patients were pretreated with fludarabine and cyclophosphamide prior to infusion of CAR-T cells. The average doses of CAR19T and CAR22T were $2.44 \pm 1.11 \times 10^6$ cells/kg and $2.19 \pm 1.22 \times 10^6$ cells/kg, respectively.

38 out of 42 (90.5%) patients achieved complete remission (CR or CRi), showing a slight improvement over the 83% complete remission rate reported for Kymriah, a CD19 CAR-T drug marketed by Novartis. 33 out of 42 (78.6%) patients achieved molecular remission and were MRD-negative by flow cytometry.

The 6-month overall survival rate and event-free survival rate were 80.0% and 60.8%, respectively.

The 12-month overall survival rate and event-free survival rate were nearly 60% and 40%, respectively.

During the follow-up period, no antigen escape of CD19 and CD22 was detected in relapsed cases after hybrid CAR-T cell therapy, but 23 patients eventually relapsed.

More than half of the patients with aggressive lymphoma achieved CR. The overall response rate exceeded 80%, higher than the 72% reported for Yescarta, a CD19 CAR-T drug marketed by Kite Pharma. The 6-month OS and EFS were approximately 90% and 60%, respectively, which were higher than the 79% and 55% reported for Yescarta.

Safety of CAR-T cell therapy:

Among patients with leukemia, the majority (88%) experienced CRS, but only 20% developed severe CRS (grade 3 or higher). 75% (47 out of 63) of the patients exhibited a significantly lower incidence of severe CRS than that reported for Kymriah, a CD19 CAR-T drug marketed by Novartis.

Among all patients with aggressive lymphoma who experienced CRS, only 10% developed severe but reversible CRS, slightly lower than the 13% reported for Yescarta, but only 17.6% developed neurotoxicity, significantly lower than the 87% reported for Yescarta.

## Example 8: Screening of exosome secretion-promoting genes

The eukaryotic expression vector selected for the experiment was pcDNA3.1 (purchased from Invitrogen). The original plasmid in the laboratory was subjected to double digestion, and a linearized vector with sticky ends was obtained after digestion with BamHI and NotI. Double digestion can prevent self-circularization of the vector. The digested product was amplified by PCR, and the size of the vector fragment was preliminarily verified by agarose gel electrophoresis, followed by gel recovery and measurement of the vector concentration. The effects of four exosome secretion-promoting genes, including Booster, CX43, KIBRA, and Rab27a, on increasing exosome production were evaluated in the present disclosure. Specifically, Booster comprises a combination of three genes (SDC4, STEAP3, and Ndab), wherein the NCBI reference sequence number for the nucleotide sequence of SDC4 is NM_002999.4, the NCBI reference sequence number for the nucleotide sequence of STEAP3 is NM_001008410.2, and the nucleotide sequence of Ndab is as shown in SEQ ID NO: 11 (the NCBI reference sequence number for the amino acid sequence of SDC4 is NP_002990.2, the NCBI reference sequence number for the amino acid sequence of STEAP3 is NP_878919.2, and the amino acid sequence of Ndab is as shown in SEQ ID NO: 12). The NCBI reference sequence number for the nucleotide sequence of CX43 is NM_000165.5 (the NCBI reference sequence number for the amino acid sequence is NP_000156.1), the NCBI reference sequence number for the nucleotide sequence of KIBRA is XM_005265853.3 (the NCBI reference sequence number for the amino acid sequence is NP_056053.1), and the NCBI reference sequence number for the nucleotide sequence of Rab27a is NM_183236.3 (the NCBI reference sequence number for the amino acid sequence is NP_899058.1).

Nucleotide sequence of Ndab (SEQ ID NO: 11):

atgaatactctccctgaacattcatgtgacgtgttgattatcggtagcggcgcagccggactttcactggcgctacgcctgg

ctgaccagcatcaggtcatcgttctaagtaaaggcccggtaacggaaggttcaacattttatgcccagggcggtattgccgccgtgtttg

atgaaactgacagcattgactcgcatgtggaagacacattgattgccggggctggtatttgcgatcgccatgcagttgaatttgtcgcca

gcaatgcacgatcctgtgtgcaatggctaatcgaccagggggtgttgtttgatacccacattcaaccgaatggcgaagaaagttaccat

ctgacccgtgaaggtggacatagtcaccgtcgtattcttcatgccgccgacgccaccggtagagaagtagaaaccacgctggtgagc

aaggcgctgaaccatccgaatattcgcgtgctggagcgcagcaacgcggttgatctgattgtttctgacaaaattggcctgccgggca

cgcgacgggttgttggcgcgtgggtatggaaccgtaataaagaaacggtggaaacctgccacgcaaaagcggtggtgctggcaacc

ggcggtgcgtcgaaggtttatcagtacaccaccaatccggatatttcttctggcgatggcattgctatggcgtggcgcgcaggctgccg

ggttgccaatctcgaatttaatcagttccaccctaccgcgctatatcacccacaggcacgcaatttcctgttaacagaagcactgcgcgg

cgaaggcgcttatctcaagcgcccggatggtacgcgttttatgcccgattttgatgagcgcggcgaactggccccgcgcgatattgtcg

cccgcgccattgaccatgaaatgaaacgcctcggcgcagattgtatgttccttgatatcagccataagcccgccgatttattcgccagc

atttcccgatgatttatgaaaagctgctcgggctggggattgatctcacacaagaaccggtaccgattgtgcctgctgcacattatacctg

cggtggtgtaatggttgatgatcatgggcgtacggacgtcgagggcttgtatgccattggcgaggtgagttataccggcttacacggcg

ctaaccgcatggcctcgaactcattgctggagtgtctggtctatggctggtcggcggcggaagatatcaccagacgtatgccttatgcc

cacgacatcagtacgttaccgccgtgggatgaaagccgcgttgagaaccctgacgaacgggtagtaattcagcataactggcacgag

ctacgtctgtttatgtgggattacgttggcattgtgcgcacaacgaagcgcctggaacgcgccctgcggcggataaccatgctccaaca

agaaatagacgaatattacgcccatttccgcgtctcaaataatttgctggagctgcgtaatctggtacaggttgccgagttgattgttcgct

gtgcaatgatgcgtaaagagagtcgggggttgcatttcacgctggattatccggaactgctcacccattccggtccgtcgatcctttccc

ccggcaatcattacataaacagataa

Amino acid sequence of Ndab (SEQ ID NO: 12):

MNTLPEHSCDVLIIGSGAAGLSLALRLADQHQVIVLSKGPVTEGSTFYAQGGI
AAVFDETDSIDSHVEDTLIAGAGICDRHAVEFVASNARSCVQWLIDQGVLFDTHIQPN
GEESYHLTREGGHSHRRILHAADATGREVETTLVSKALNHPNIRVLERSNAVDLIVSD
KIGLPGTRRVVGAWVWNRNKETVETCHAKAVVLATGGASKVYQYTTNPDISSGDGI
AMAWRAGCRVANLEFNQFHPTALYHPQARNFLLTEALRGEGAYLKRPDGTRFMPDF
DERGELAPRDIVARAIDHEMKRLGADCMFLDISHKPADFIRQHFPMIYEKLLGLGIDL
TQEPVPIVPAAHYTCGGVMVDDHGRTDVEGLYAIGEVSYTGLHGANRMASNSLLEC
LVYGWSAAEDITRRMPYAHDISTLPPWDESRVENPDERVVIQHNWHELRLFMWDYV
GIVRTTKRLERALRRITMLQQEIDEYYAHFRVSNNLLELRNLVQVAELIVRCAMMRKE
SRGLHFTLDYPELLTHSGPSILSPGNHYINR

Common methods for cell transfection include artificial liposome method, viral transfection, and PEI transfection. Since artificial liposomes are relatively expensive and viral transfection may cause a certain degree of damage to HEK293T or HEK293F cells used in the experiment, PEI transfection is selected in the present disclosure. DNA can be transfected into host cells using polyethylenimine (PEI), which is a stable cationic polymer. PEI condenses DNA into positively charged particles that bind to the anionic cell surface. Therefore, the DNA-PEI complex is endocytosed by cells, and the DNA is released into the cytoplasm. PEI is superior to other cell transfection reagents due to experimental cost considerations. PEI transfection imposes high requirements on cell viability, number of cells, and survival rate. HEK293T or HEK293F cells pre-cultured for 24 hours were examined under a microscope. When the cell confluence reaches 60% to 70%, the cells are in the logarithmic growth phase, which represents the optimal period for cell transfection, ensuring good cell viability with a survival rate of 90% or more. Typically, 1 $\mu$g of DNA was used for transfection per 1 mL of culture. PEI and DNA should be diluted to 1/20 of the total culture volume separately before mixing. 30 $\mu$L of pre-prepared PEI (1 $\mu$g/$\mu$L) was added to 100 $\mu$L of pure DMEM medium in a 1.5 mL centrifuge tube and vortex-mixed. Subsequently, 275 ng of CD63-NanoLuc plasmid (purchased from Wuhan Miaoling Biotechnology Co., Ltd.) and 725 ng of the aforementioned constructed exosome secretion-promoting plasmid (mass ratio of 1:3) were added to a new 1.5 mL centrifuge tube containing 100 $\mu$L of pure DMEM medium, vortex-mixed, and allowed to stand for 5 minutes. The incubated PEI was added to a centrifuge tube containing the plasmid mixture, wherein a mass ratio of 1:3 between PEI and plasmid was recommended. The mixture was allowed to stand for 30 minutes to ensure complete encapsulation of the double plasmid by PEI, thus forming a PEI-DNA complex. CD63-NanoLuc was used for exosome quantification, and equal amounts of each exosome secretion-promoting plasmid were ensured. A blank control was set up by adding the same concentration of CD63-NanoLuc and an empty vector (pcDNA3.1). Finally, the PEI-DNA mixture was added dropwise to the medium containing monolayer cells as gently as possible to avoid detaching the adherent cells. The dish was gently shaken to mix and incubated in a cell incubator at 37°C with 5% $CO_2$. After 24 hours of incubation, the plasmid had been transfected into the cells, and 10 mL of DMEM complete medium was added for replenishment to ensure good culture conditions. The cells were then incubated again in a cell incubator at 37°C with 5% $CO_2$.

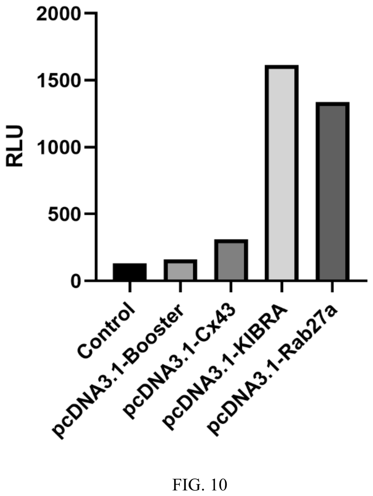

As shown in FIG. 10, the results of exosome purification and quantification demonstrated that KIBRA and Rab27a significantly increased exosome production.

**Example 9: Collection of exosomes Exo-CAR-CD19 and Exo-CAR-CD22**

(I) Construction of HEK-293T or HEK-293F cells transfected with recombinant lentivirus LVCAR19 or LVCAR22 + exosome secretion-promoting plasmid + granzyme and perforin overexpression plasmids

(i) Plasmid used for transfection in this example and construction method thereof

(1) Recombinant lentivirus LVCAR19 or LVCAR22, wherein the construction method refers to Examples 1 to 3 of the present disclosure.

(2) Exosome secretion-promoting plasmid, wherein the construction method refers to Example 8 of the present disclosure, and the exosome secretion-promoting gene in the exosome secretion-promoting plasmid used in this example is KIBRA. The exosome secretion-promoting gene KIBRA was purchased from Wuhan Miaoling Biotechnology Co., Ltd. or obtained by RT-PCR in cells. The vector was prepared by digesting pcDNA3.1 with NheI and XhoI, followed by homologous recombination, and verified by Sanger sequencing at Sangon Biotech.

(3) Construction of perforin overexpression plasmid pcDNA3.1-CD63-PRF1 fused with CD63 and granzyme overexpression plasmid pcDNA3.1-CD63-GZMB fused with CD63: Plasmid CD63-NanoLuc was digested with restriction enzymes EcoRI and AgeI at 37°C to obtain the sequences of CD63 (the nucleotide sequence is as shown in SEQ ID NO: 5, and the amino acid sequence is as shown in SEQ ID NO: 6). The nucleotide sequences of perforin PRF1 and granzyme GZMB are as shown in SEQ ID NO: 7 and SEQ ID NO: 9, respectively, and the amino acid sequences are as shown in SEQ ID NO: 8 and SEQ ID NO: 10, respectively. The vector was prepared by digesting pcDNA3.1 with NheI and XhoI, followed by homologous recombination, and verified by Sanger sequencing at Sangon Biotech.

Nucleotide sequence of CD63 (SEQ ID NO: 5):

atggcggtggaaggaggaatgaaatgtgtgaagttcttgctctacgtcctcctgctggcctttttgcgcctgtgcagtgggga

ctgattgccgtgggtgtcggggcacagcttgtcctgagtcagaccataatccaggggggctacccctggctctctgttgccagtggtcat

catcgcagtgggtgtcttcctcttcctggtggctttttgtgggctgctgcgggggcctgcaaggagaactattgtcttatgatcacgtttgcca

tctttctgtctcttatcatgttggtggaggtggccgcagccattgctggctatgtgtttagagataaggtgatgtcagagtttaataacaactt

ccggcagcagatggagaattacccgaaaaataaccacactgcttcgatcctggacaggatgcaggcagatttttaagtgctgtggggct

gctaactacacagattgggagaaaatcccttccatgtcgaagaaccgagtccccgactcctgctgcattaatgttactgtgggctgtggg

attaatttcaacgagaaggcgatccataaggagggctgtgtggagaagattgggggctggctgaggaaaaatgtgctggtggtagct

gcagcagcccttggaattgcttttgtcgaggtttttgggaattgtctttgcctgctgcctcgtgaagagtatcagaagtggctacgaggtga

tg

Amino acid sequence of CD63 (SEQ ID NO: 6):

MAVEGGMKCVKFLLYVLLLAFCACAVGLIAVGVGAQLVLSQTIIQGATPGSL

LPVVIIAVGVFLFLVAFVGCCGACKENYCLMITFAIFLSLIMLVEVAAAIAGYVFRDKV

MSEFNNNFRQQMENYPKNNHTASILDRMQADFKCCGAANYTDWEKIPSMSKNRVP

DSCCINVTVGCGINFNEKAIHKEGCVEKIGGWLRKNVLVVAAAALGIAFVEVLGIVFA

CCLVKSIRSGYEVM

Nucleotide sequence of perforin PRF1 (SEQ ID NO: 7):

atggcagcccgtctgctcctcctgggcatccttctcctgctgctgcccctgcccgtccctgccccgtgccacacagccgc

acgctcagagtgcaagcgcagccacaagttcgtgcctggtgcatggctggccgggggagggtgtggacgtgaccagcctccgccgc

tcgggctccttcccagtggacacacaaaggttcctgcggcccgacggcacctgcaccctctgtgaaaatgccctacaggagggcacc

ctccagcgcctgcctctggcgctcaccaactggcgggcccagggctctggctgccagcgccatgtaaccagggccaaagtcagctc

cactgaagctgtggcccgggatgcggctcgtagcatccgcaacgactggaaggtcgggctggacgtgactcctaagcccaccagca

atgtgcatgtgtctgtggccggctcacactcacaggcagccaactttgcagcccagaagacccaccaggaccagtacagcttcagca

ctgacacggtggagtgccgcttctacagtttccatgtggtacacactcccccgctgcaccctgacttcaagagggccctcggggacct

gccccaccacttcaacgcctccacccagcccgcctacctcaggcttatctccaactacggcacccacttcatccgggctgtggagctg

ggtggccgcatatcggccctcactgccctgcgcacctgcgagctggccctggaagggctcacggacaacgaggtggaggactgcc

tgactgtcgaggcccaggtcaacataggcatccacggcagcatctctgccgaagccaaggcctgtgaggagaagaagaagaagca

caagatgacggcctccttccaccaaacctaccgggagcgccattcggaagtggttggcggccatcacacctccattaacgacctgctg

ttcgggatccaggccgggccccgagcagtactcagcctgggtaaactcgctgcccggcagccctggcctggtggactacaccctgga

acccctgcacgtgctgctggacagccaggacccgcggcgggaggcactgaggagggccctgagtcagtacctgacggacagggc

tcgctggagggactgcagccggccgtgcccaccagggcggcagaagagcccccgagacccatgccagtgtgtgtgccatggctca

gcggtcaccacccaggactgctgccctcggcagagggggcctggcccagctggaggtgaccttcatccaagcatggggcctgtggg

gggactggttcactgccacggatgcctatgtgaagctcttctttggtggccaggagctgaggacgagcaccgtgtgggacaataacaa

ccccatctggtcagtgcggctggattttggggatgtgctcctggccacagggggggcccctgaggttgcaggtctgggatcaggactct

ggcagggacgatgacctccttggcacctgtgatcaggctcccaagtctggttcccatgaggtgagatgcaacctgaatcatggccacc

taaaattccgctatcatgccaggtgcttgccccacctgggaggaggcacctgcctggactatgtcccccaaatgcttctgggggagcct

ccaggaaaccggagtggggccgtgtgg

Amino acid sequence of perforin PRF1 (SEQ ID NO: 8):

MAARLLLLLGILLLLLLPLPVPAPCHTAARSECKRSHKFVPGAWLAGEGVDVTS
LRRSGSFPVDTQRFLRPDGTCTLCENALQEGTLQRLPLALTNWRAQGSGCQRHVTRA
KVSSTEAVARDAARSIRNDWKVGLDVTPKPTSNVHVSVAGSHSQAANFAAQKTHQD

QYSFSTDTVECRFYSFHVVHTPPLHPDFKRALGDLPHHFNASTQPAYLRLISNYGTHFI
RAVELGGRISALTALRTCELALEGLTDNEVEDCLTVEAQVNIGIHGSISAEAKACEEKK
KKHKMTASFHQTYRERHSEVVGGHHTSINDLLFGIQAGPEQYSAWVNSLPGSPGLVD
YTLEPLHVLLDSQDPRREALRRALSQYLTDRARWRDCSRPCPPGRQKSPRDPCQCVC
HGSAVTTQDCCPRQRGLAQLEVTFIQAWGLWGDWFTATDAYVKLFFGGQELRTSTV
WDNNNPIWSVRLDFGDVLLATGGPLRLQVWDQDSGRDDDLLGTCDQAPKSGSHEV
RCNLNHGHLKFRYHARCLPHLGGGTCLDYVPQMLLGEPPGNRSGAVW

Nucleotide sequence of granzyme GZMB (SEQ ID NO: 9):

atgcaaccaatcctgcttctgctggccttcctcctgctgcccagggcagatgcaggggagatcatcggggggacatgagg
ccaagcccccactcccgcccctacatggcttatcttatgatctgggatcagaagtctctgaagaggtgcggtggcttcctgatacaagac
gacttcgtgctgacagctgctcactgttggggaagctccataaatgtcaccttggggggcccacaatatcaaagaacaggagccgaccc
agcagtttatccctgtgaaaagacccatcccccatccagcctataatcctaagaacttctccaacgacatcatgctactgcagctggaga
gaaaggccaagcggaccagagctgtgcagcccctcaggctacctagcaacaaggcccaggtgaagccagggcagacatgcagtg
tggccggctggggggcagacggcccccctgggaaaacactcacacacactacaagaggtgaagatgacagtgcaggaagatcgaa
agtgcgaatctgacttacgccattattacgacagtaccattgagttgtgcgtgggggacccagagattaaaaagacttcctttaaggggg
actctggaggccctcttgtgtgtaacaaggtggcccagggcattgtctcctatggacgaaacaatggcatgcctccacgagcctgcac
caaagtctcaagctttgtacactggataaagaaaaccatgaaacgccactaa

Amino acid sequence of granzyme GZMB (SEQ ID NO: 10):

MQPILLLLAFLLLPRADAGEIIGGHEAKPHSRPYMAYLMIWDQKSLKRCGGF
LIQDDFVLTAAHCWGSSINVTLGAHNIKEQEPTQQFIPVKRPIPHPAYNPKNFSNDIML
LQLERKAKRTRAVQPLRLPSNKAQVKPGQTCSVAGWGQTAPLGKHSHTLQEVKMTV
QEDRKCESDLRHYYDSTIELCVGDPEIKKTSFKGDSGGPLVCNKVAQGIVSYGRNNG
MPPRACTKVSSFVHWIKKTMKRH

(ii) Cell culture and transfection

HEK-293T cells (DSMZ: ACC-635) were cultured in DMEM (Gibco) supplemented with 10% (v/v) fetal bovine serum (FBS, VIVA) and 1% (v/v) penicillin/streptomycin solution (Gibco). HEK-293F cells were cultured in F medium, which was prepared by mixing SMM 293-TII medium with OPM-293 CD05 medium at an equal ratio, in a humidified atmosphere containing 5% $CO_2$. For continuous passaging of these cells, 0.05% trypsin-EDTA (Gibco) was used.

Transfection (HEK-293T, HEK-293F): $5 \times 10^6$ cells were inoculated into a 10 cm cell culture dish 24 hours before transfection. 30 $\mu$L of PEI (PEI, 20000 MW, Polysciences; stock solution: 1 mg/mL in dH) was incubated with 10 $\mu$g of total DNA ((i) recombinant lentivirus LVCAR19 or LVCAR22; (ii) exosome secretion-promoting plasmid KIBRA; (iii) pcDNA3.1-CD63-PRF1; (iv) pcDNA3.1-CD63-GZMB) for co-transfection into HEK-293T or HEK-293F cells in the 10 cm cell culture dish. 16 hours after transfection, the medium was replaced with fresh DMEM.

(II) Collection of exosomes from the cells constructed in step (I) of this example

HEK-293F cells transfected with recombinant lentivirus LVCAR19 or LVCAR22 + exosome secretion-promoting plasmid KIBRA + granzyme and perforin overexpression plasmids obtained in step (I) were cultured in conditioned medium (CM), and then exosomes were collected by the following steps:

1. The CM (conditioned medium) was pre-cleared by differential centrifugation and filtration (starting from step 2) as follows.

(1) The CM was centrifuged at 500×g for 5 minutes at 4°C, the supernatant was transferred to a new tube, and the pellet was discarded. The centrifugation step was repeated once. The supernatant was collected, and the pellet was discarded.
(2) The supernatant from step (1) was centrifuged at 2,000×g for 15 minutes at 4°C, and the pellet was discarded. The collected supernatant was filtered once through a 0.22 $\mu$m filter.

2. During the pre-clearing process, a 25% (w/w) sucrose solution in deuterium oxide was prepared by accurately weighing 1.9 g ($\pm$0.001 g) of sucrose in a universal tube, followed by filling with deuterium oxide until the weight reached 7.6 g ($\pm$0.001 g).

3. The ultracentrifuge tube was filled with 22.5 mL of pre-cleared CM. If the current volume was less than 22.5 mL, the CM was supplemented to 22.5 mL with 0.22 μm filtered PBS.

4. A glass pipette was placed in the centrifuge tube, and 3 mL of sucrose solution was added by the pipette to form a separate layer beneath the CM.

5. The centrifuge tube containing the layered CM/sucrose solution was carefully transferred to the bucket of a swing-out rotor, and the bucket was secured into the rotor.

6. The rotor was placed in an ultracentrifuge and centrifuged at 100,000×g for 1.5 hours at 4°C.

7. 2 mL of the sucrose layer was collected and added to an ultracentrifuge tube containing 20 mL of filtered PBS for washing.

8. The tube was placed in a fixed-angle rotor and centrifuged at 100,000×g for 1.5 hours at 4°C.

9. The supernatant was carefully removed using a 10 mL pipette, and the pellet was resuspended in 400 μL of filtered PBS.

10. The exosomes were stored at 4°C for short-term storage or at -80°C for long-term storage for subsequent experimental use.

**Example 10: Identification and phenotypic analysis of exosomes Exo-CAR-CD19 and Exo-CAR-CD22**

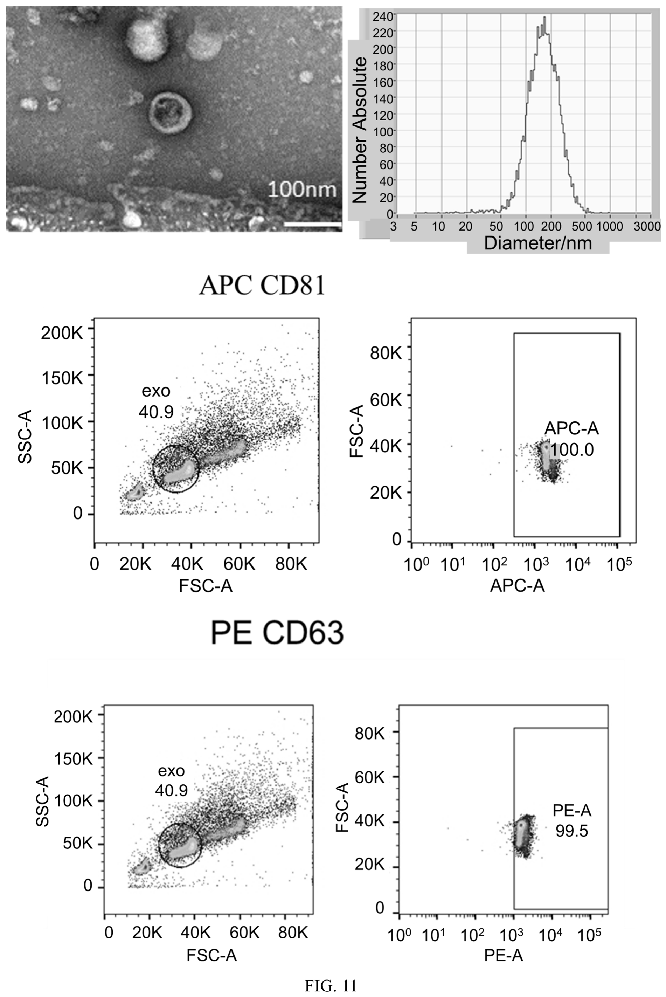

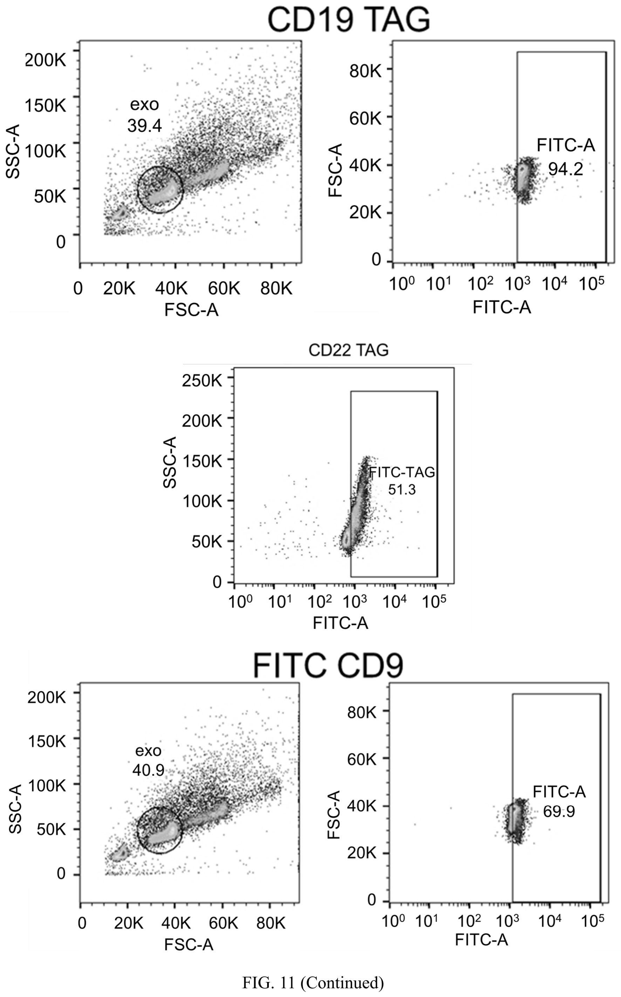

The Exo-CAR-CD19 and Exo-CAR-CD22 collected from Example 9 were subjected to electron microscopy, particle size, and flow cytometry analysis, and the results are shown in FIG. 11.

10.1 Transmission electron microscopy (TEM) assay of exosomes/TEM procedure

1. Exosomes were fixed with glutaraldehyde.

2. 5 to 10 μL of exosome solution was added dropwise onto a copper grid, adsorbed at room temperature for approximately 10 minutes, and excess liquid was carefully removed with filter paper. 10 μL of 2% phosphotungstic acid solution (pH = 6.5) was then added dropwise onto the copper grid, and the exosomes were stained at room temperature for 2 minutes. Excess staining solution was carefully removed with filter paper, and the copper grid was air-dried at room temperature.

3. TEM observation was performed at a voltage of 120 kV.

10.2 Nano-flow cytometry

Monoclonal antibodies (mAbs, purchased from Biolegend) recognizing exosomal protein markers (CD9, CD63, and CD81) were stained with phycoerythrin (PE, purchased from Biolegend). Nano-flow cytometry enabled the counting of exosomes at the single-particle level, as well as quantitative analysis of single or dual protein markers, either separately or in combination, on the exosomal membrane surface. The specific steps were as follows:

1. Preparation of enhancer-containing washing buffer (purchased from Wako, Code No. 297-79701)

(1) 10× washing buffer was diluted to 1× with ultrapure water for use.

(2) 100× binding enhancer was added to the 1× washing buffer at a ratio of 1:100 to obtain an enhancer-containing washing buffer.

2. Pretreatment of magnetic beads

(1) The required amount of magnetic bead suspension was added at 10 μL per tube, and 100 μL of enhancer-containing washing buffer was added per 10 μL of magnetic beads (purchased from Wako, Code No. 297-79701). The tube was vortexed for 5 seconds, rapidly centrifuged, allowed to stand on a magnetic rack for 1 minute, and the supernatant was carefully removed.

(2) The above steps were repeated once.

3. Exosome binding

(1) 100 μL of enriched exosome suspension was taken.

(2) 100 μL of exosome suspension was added to an EP tube containing magnetic beads, followed by vortexing for 5 seconds.

(3) 1 μL of exosome binding enhancer (100×) was added to the EP tube, followed by vortexing for 5 seconds.

(4) The EP tube was placed at room temperature for 1 hour and vortexed every 20 minutes to fully capture exosomes.

(5) The EP tube was rapidly centrifuged and allowed to stand on a magnetic rack for 1 minute, and the supernatant was carefully removed. 500 $\mu$L of binding enhancer-containing washing buffer was added to the EP tube, followed by vortexing for 5 seconds. The EP tube was rapidly centrifuged and placed on the magnetic rack for 1 minute, and the supernatant was removed.

(6) The above steps were repeated once.

(7) A corresponding volume of binding enhancer-containing washing buffer was added to the EP tube at 100 $\mu$L per tube, followed by vortexing for 3 seconds to obtain an exosome-containing magnetic bead suspension.

4. Exosome immunostaining

(1) 100 $\mu$L of exosome-containing magnetic bead suspension was added to each EP tube.

(2) A labeled anti-exosome surface marker protein antibody and a labeled isotype control antibody were added to the EP tube, followed by vortexing for 5 seconds.

(3) The EP tube was placed at room temperature for 1 hour and vortexed every 20 minutes to allow antibody binding.

(4) The incubated EP tube was rapidly centrifuged and placed on a magnetic rack for 1 minute. After the magnetic beads and supernatant were separated, the supernatant was removed.

(5) 300 $\mu$L of washing buffer (enhancer-containing) was added to each EP tube, followed by vortexing for 5 seconds. The EP tube was rapidly centrifuged and placed on the magnetic rack for 1 minute. After the magnetic beads and supernatant were separated, the supernatant was removed.

(6) The above steps were repeated once.

(7) 300 $\mu$L of washing buffer (enhancer-containing) was added to each EP tube, followed by vortexing for 5 seconds for flow cytometry analysis.

The exosome phenotype was determined by flow cytometry, and the results demonstrated that 94.2% of the exosomes contained CAR molecular structures, while all of the exosomes expressed the characteristic exosomal proteins CD81, CD63, and CD9.

10.3 NTA (for exosome samples, particle size distribution and concentration were measured): (i) The sample cell was washed with deionized water; (ii) The instrument was calibrated with polystyrene microspheres (100 nm); (iii) The sample cell was washed with 1$\times$ PBS buffer (Biological Industries, Israel); (iv) The sample was diluted with 1$\times$ PBS buffer (BI, Israel) and injected for measurement. The results demonstrated that the diameter distribution of exosomes was mainly concentrated around 150 nm.

**Example 11: Determination of granzyme and perforin in 293F engineered exosomes**

11.1 The culture supernatant of Exo-CAR cell killing assay was centrifuged at 1,000$\times$g for 10 minutes at 4°C, aliquoted into 200 $\mu$L per EP tube, and stored in a freezer at -80°C to avoid repeated freeze-thaw cycles.

11.2 The experiment was conducted by referring to the instructions of two ELISA kits (Human Perforin ELISA Kit, Solarbio, Cat. No.: SEKH-0295; Human Granzyme ELISA Kit, Solarbio, Cat. No.: SEKH-0193). Since the procedures and reagents were mostly identical, the Human Perforin ELISA Kit was taken as an example herein.

11.2.1 Reagent equilibration: 30 minutes before the experiment, the kit and samples to be tested were placed at room temperature. If crystallization occurs, the samples are placed in a water bath at 37°C until completely dissolved.

11.2.2 Preparation of washing buffer: The 20$\times$ concentrated washing buffer was diluted with deionized water to prepare a 1$\times$ working washing buffer, wherein the volume of the working washing buffer was 300 mL (plus 30 mL). The washing buffer is universal. If the two kits are used together, 560 mL (plus 20 mL) is prepared.

11.2.3 Serial dilution of standard (inconsistency between the two kits): The standard (SR1) (0.5 mL of perforin, 1 mL of granzyme) was added to the lyophilized standard, allowed to stand for 15 minutes until completely dissolved, and then gently mixed. The mixture was serially diluted according to the instructions. The reconstituted standard stock solution can be stored at -20 or -80°C.

11.2.4 Preparation of biotinylated antibody working solution: The 100$\times$ antibody concentrate was diluted with the assay diluent (SR2) to prepare a 1$\times$ working solution (thoroughly mixed before dilution), which was added to the reaction wells within 30 minutes. 6500 $\mu$L of working solution (65 $\mu$L + 6435 $\mu$L) was prepared.

11.2.5 Preparation of enzyme conjugate working solution (inconsistency between the two kits): The enzyme conjugate was diluted with the enzyme conjugate diluent (SR3) to prepare a 1$\times$ working solution (centrifuged before dilution) (granzyme 40$\times$, perforin 100$\times$), which was added to the reaction wells within 30 minutes. For perforin, 6500 $\mu$L of working solution (65 $\mu$L + 6435 $\mu$L) was prepared. For granzyme B, 6000 $\mu$L of working solution (150 $\mu$L + 5850 $\mu$L) was prepared.

11.2.6 Automatic plate washing settings: The liquid in the wells of an ELISA plate was completely removed by flicking. The plate was dried by blotting on absorbent paper, injected with 300 μL of washing buffer, with an interval of 30 seconds between injection and aspiration, and washed 5 times.

11.2.7 Test procedure:

Test procedure: Granzyme B

1. 30 minutes before the experiment, the Human Granzyme ELISA Kit was taken out and allowed to reach room temperature. Before adding the standard/sample, the plate was washed 3 times and dried by flicking.
2. 100 μL of standard and test samples were added to the reaction wells. The plate was sealed and incubated at 37°C for 90 minutes.
The plate was tapped and washed 4 times.
3. 10 μL of biotinylated antibody working solution was added to the reaction wells. The plate was sealed and incubated at 37°C for 60 minutes.
The plate was tapped and washed 4 times.
4. 100 μL of enzyme conjugate working solution was added to the reaction wells. The plate was sealed and incubated at 37°C for 30 minutes.
The plate was tapped and washed 5 times.
5. 100 μL of chromogenic substrate was added to the reaction wells. The plate was sealed and incubated at 37°C in the dark for 15 minutes for color development.
6. 50 μL of stop solution was added, and the OD value at 450 nm was immediately measured using a microplate reader (within 5 minutes).

Test procedure: Perforin

1. 30 minutes before the experiment, the Human Perforin ELISA Kit was taken out and allowed to reach room temperature. Before adding the standard/sample, the plate was washed 3 times and dried by flicking.
2. 100 μL of standard and test samples were added to the reaction wells. The plate was sealed and incubated with shaking at room temperature (25±2°C) for 120 minutes.
The plate was tapped and washed 4 times.
3. 100 μL of biotinylated antibody working solution was added to the reaction wells. The plate was sealed and incubated with shaking at room temperature (25±2°C) for 60 minutes.
The plate was tapped and washed 4 times.
4. 100 μL of enzyme conjugate working solution was added to the reaction wells. The plate was sealed and incubated with shaking at room temperature (25±2°C) for 60 minutes.
The plate was tapped and washed 5 times.
5. 100 μL of chromogenic substrate was added to the reaction wells. The plate was sealed and incubated at room temperature (25±2°C) in the dark for 10 to 30 minutes for color development.
6. 50 μL of stop solution was added, and the OD value at 450 nm was immediately measured using a microplate reader (within 5 minutes).

Note: The plate was shaken at 300 to 400 rpm using a micro oscillator (preferably without splashing of the liquid in micro wells).

Judgment of results:

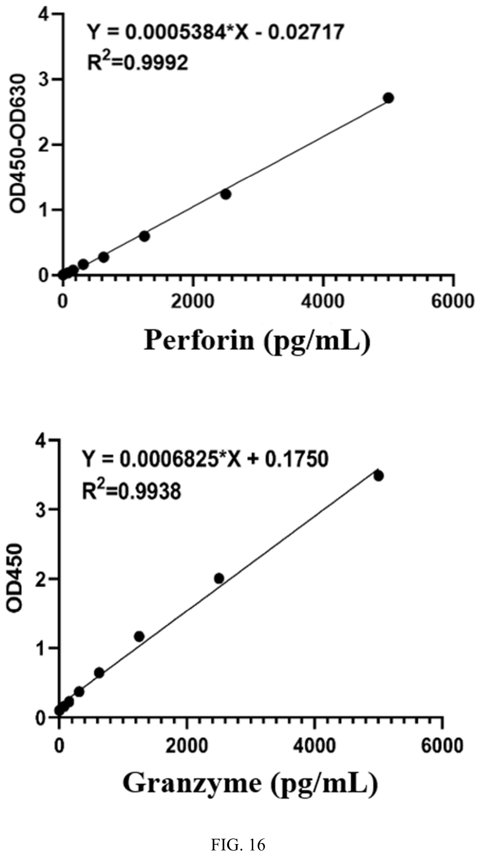

1. The OD value at 450 nm was measured using a microplate reader. Dual-wavelength spectrophotometry was selected with a reference wavelength of 630 nm. If dual-wavelength spectrophotometry cannot be performed, the OD measurement at 630 nm shall be subtracted from the OD measurement at 450 nm.
2. Calculating the average OD value of the standard and sample: The OD value of the zero well shall be subtracted from the OD value of each standard and sample.
3. With the concentration of the standard as the abscissa and the absorbance (OD value) as the ordinate, a standard curve was plotted using software (as shown in FIG. 16). The granzyme B levels in the sample can be calculated from the corresponding OD value based on the standard curve.

4. If the OD value of the sample exceeds the upper limit of the standard curve, the sample should be appropriately diluted and retested, and the dilution factor should be multiplied when calculating the concentration.

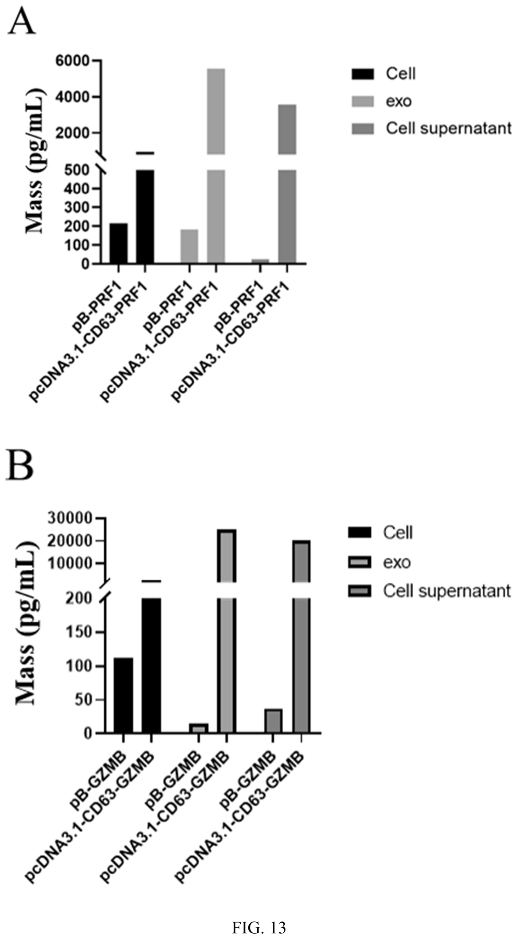

The test results are shown in FIG. 13. Exosomes from the following groups were collected for quantification of granzyme and perforin: pB-PRF1 represents the perforin co-expression plasmid (backbone pcDNA3.1); pcDNA3.1-CD63-PRF1 represents the perforin plasmid fused with CD63; pB-GZMB represents the granzyme expression plasmid (backbone pcDNA3.1); pcDNA3.1-CD63-GZMB represents the granzyme plasmid fused with CD63; Cell, exo, and Cell supernatant represent the granzyme and perforin levels in transfected cells, exosomes, and cell culture supernatant, respectively. The results demonstrated that granzyme and perforin were enriched in exosomes, reaching 4700 pg/mL and 25000 pg/mL, respectively, which were significantly higher than their expression levels in cells. When cells were not transfected with plasmids containing granzyme or perforin genes, the granzyme and perforin levels in the cells, exosomes, and cell culture supernatant were 0 pg/mL.

The granzyme and perforin levels in the engineered exosomes of the present disclosure compared to the exosomes isolated from CAR19T and CAR22T are shown in Table 7 below.

Table 7

| Type | Perforin (pg/mL) | Granzyme (pg/mL) |
| --- | --- | --- |
| CD19 exosome | 25379.5 | 6189.4 |
| CAR-T19 exosome | 26213.8 | 7246.2 |
| CD22 exosome | 27216.1 | 8517.6 |
| CAR-T22 exosome | 8053.5 | 3298.4 |

As shown in Table 7, the CD22 engineered exosomes of the present disclosure exhibited significantly higher perforin and granzyme levels compared to the exosomes isolated from CAR22T; whereas the CD19 engineered exosomes of the present disclosure achieved perforin and granzyme levels substantially comparable to those of the exosomes isolated from CAR19T.

**Example 12: *In vivo* anti-tumor efficacy of exosomes Exo-CAR-CD19 and Exo-CAR-CD22**

To evaluate the anti-tumor efficacy of Exo-CAR *in vivo,* immunodeficient (NOD-SCID) mice and Raji-luc cells were selected to establish a tumor model. After successful modeling, Exo-CAR was injected, and the experimental results were detected using an IVIS small animal *in vivo* imaging system (Xenogen, Hopkinton, USA). The experimental steps were as follows:

1. Modeling: 5-week-old NOD-SCID mice were subcutaneously injected with Raji-luc cells at $5\times10^6$ cells/mouse.
2. After 7 days, the mice were imaged by intraperitoneal injection of D-luciferin (Molecular Imaging Products, Bend, USA) at a dose of 150 mg/kg, followed by anesthesia by intraperitoneal injection of sodium pentobarbital at a dose of 75 mg/kg. After 10 minutes, the optical signals were collected using the IVIS small animal *in vivo* imaging system (Xenogen, Hopkinton, USA).
3. On the day of successful modeling, Exo-CAR was injected, with the following groups:

(1) CD19 group, injected with Exo-CAR-CD19 at $5\times10^{12}$ cells/mouse;
(2) CD22 group, injected with Exo-CAR-CD22 at $5\times10^{12}$ cells/mouse;
(3) CD19+CD22 group, first injected with Exo-CAR-CD19 at $5\times10^{12}$ cells/mouse, followed by injection with Exo-CAR-CD22 at $5\times10^{12}$ cells/mouse 3 days later;
(4) CD22+CD19 group: first injected with Exo-CAR-CD22 at $5\times10^{12}$ cells/mouse, followed by injection with Exo-CAR-CD19 at $5\times10^{12}$ cells/mouse 3 days later;
(5) NT group, negative control exosomes;
(6) EGFP group, non-target control exosomes.

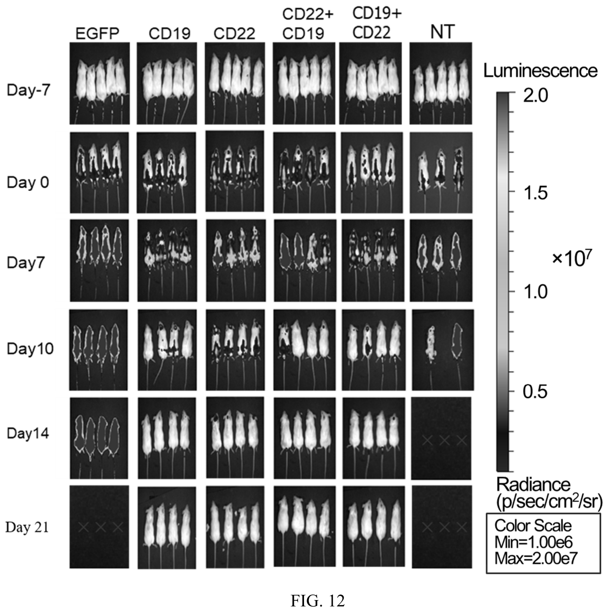

4. Imaging was performed 21 days after the injection of Exo-CAR, and the experimental results were analyzed, as shown in FIG. 12. The results demonstrated that compared to the NT group and the EGFP group, the tumor burden in the CAR19 group, the CAR22 group, the CD19+CD22 group, and the CD22+CD19 group was significantly reduced, and the tumor was completely eradicated on Day 21, indicating that Exo-CAR exhibited anti-tumor efficacy *in vivo.*

**Example 13: Killing effect of engineered exosomes on tumor cells**

Cell killing analyzed by dual fluorescence flow cytometry

Dual fluorescence flow cytometry is a method for analyzing the killing effect by labeling target cells with two different fluorescent dyes before co-culturing with effector cells, in which the reduction of double-fluorescent cells is measured on a flow cytometer to determine the dead cells and calculate the killing efficiency.

The specific method was as follows:

(1) Cells expressing EGFP were used as target cells and resuspended in medium containing NK-EV.
(2) The suspended cells were inoculated into a 96-well plate at a density of $2\times10^4$ cells per well.
(3) The experimental group was supplemented with 400 $\mu$g of exosomes, with a final volume of 100 $\mu$L per well.
(4) The positive control group was not supplemented with exosomes.
(5) After all the wells were mixed, the plate was incubated in a $CO_2$ incubator at 37°C for approximately 12 hours or 24 hours.
(6) After incubation, the cells can be directly detected by flow cytometry.

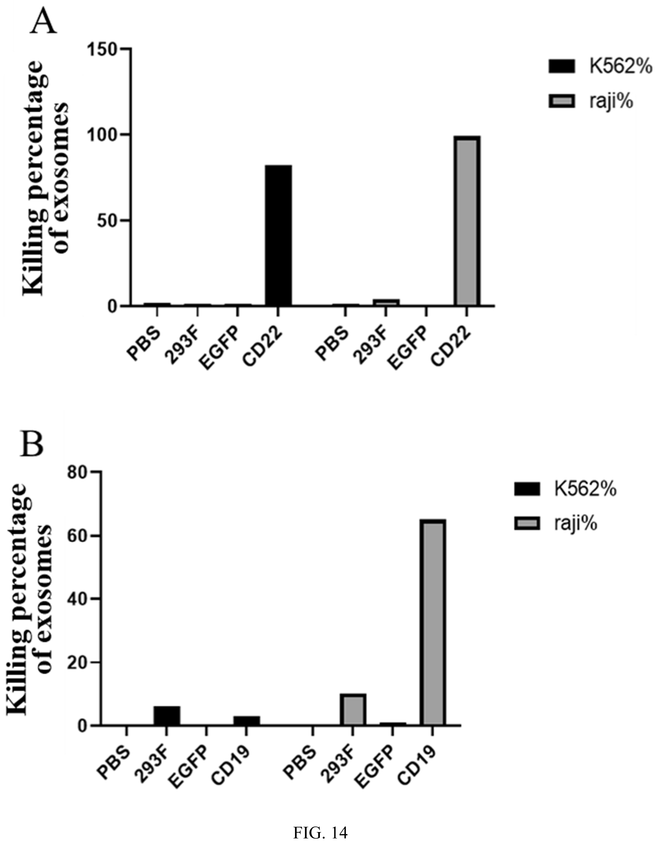

The results are shown in FIG. 14. Cell killing was analyzed in a PBS control group, a 293F cell-derived exosome group, an EGFP CAR exosome control group, an Anti-CD19 CAR exosome group, and an Anti-CD22 CAR exosome group. The target cells were RAJI-EGFP-LUC, and the negative control cells were K562-EGFP-LUC. The killing results demonstrated that the Anti-CD19 CAR exosome group and the Anti-CD22 CAR exosome group exhibited killing rates of 63% and 98%, respectively, against the positive target cells.

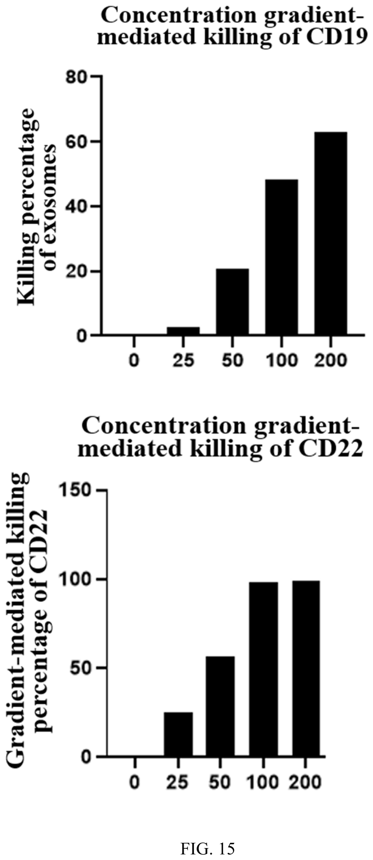

Cell killing was analyzed using the same dual fluorescence flow cytometry described above, wherein in step (3), an exosome gradient of 0 $\mu$g to 200 $\mu$g was set. The results are shown in FIG. 15, indicating that the killing by Anti-CD19 CAR exosomes and Anti-CD22 CAR exosomes was dose-dependent. If the dose is sufficiently large, e.g., greater than 200 $\mu$g, the killing percentage of CAR-T can be reached or even exceeded.

**Example 14: Exosome secretion-promoting gene CX43 (S368A)**

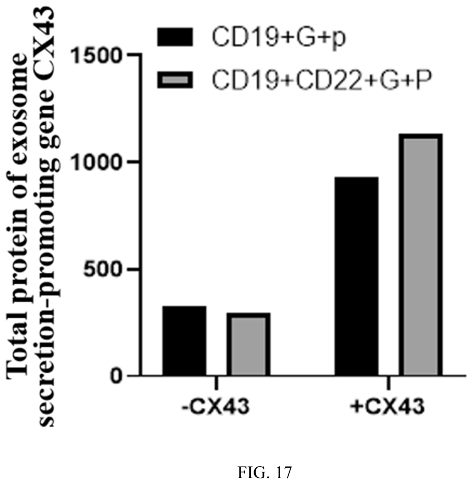

HEK-293T or HEK-293F cells transfected with recombinant lentivirus LVCAR19 or LVCAR22 + exosome secretion-promoting plasmid + granzyme and perforin overexpression plasmids were constructed using a method similar to that in Example 9, with the difference being that the exosome secretion-promoting gene in the exosome secretion-promoting plasmid used in this example is CX43 (S368A), wherein the NCBI reference sequence number for the amino acid sequence of CX43 is NP_000156.1, with S replaced by A at position 368 (the NCBI reference sequence number for the nucleotide sequence encoding CX43 is NM_000165.5, with AGC replaced by GCC at positions 1317 to 1319). The total protein secreted by the constructed cells was detected, and the results are shown in FIG. 17. Through preliminary optimization of plasmid ratios, including co-transfection of the secretion-promoting plasmid CX43 (S368A), the secretion of CAR-CD19 and CAR-CD22 engineered exosomes specifically loaded with granzyme and perforin was increased by 3 to 4 times, indicating potential for further improvement in large-scale exosome production.

The examples described above are merely preferred examples provided to fully illustrate the present disclosure, and the scope of protection of the present disclosure is not limited thereto. Any equivalent substitutions or modifications made by those skilled in the art based on the present disclosure shall fall within the scope of protection of the present disclosure. The scope of protection of the present disclosure is defined by the claims.

**Claims**

1. A CAR molecule, wherein the CAR molecule is a CAR molecule targeting CD19 and/or a CAR molecule targeting CD22; the amino acid sequence of the CAR molecule targeting CD19 is as shown in SEQ ID NO: 1, and the amino acid sequence of the CAR molecule targeting CD22 is as shown in SEQ ID NO: 2.

2. An isolated nucleic acid, wherein the nucleic acid encodes the CAR molecule according to claim 1; preferably, the nucleotide sequence encoding the CAR molecule targeting CD19 is as shown in SEQ ID NO: 3, and/or the nucleotide sequence encoding the CAR molecule targeting CD22 is as shown in SEQ ID NO: 4.

3. A recombinant expression vector, wherein the recombinant expression vector comprises the nucleic acid according to claim 2;

    preferably, the recombinant expression vector is a lentiviral vector;
    more preferably, the backbone of the lentiviral vector is pLVX-EF1α-IRES-Puro.

4. A transformant, wherein the transformant expresses the CAR molecule according to claim 1;

    preferably, the transformant is a host cell transfected with the recombinant expression vector according to claim 3;
    more preferably, the host cell is a mammalian cell, such as a T cell, a 293 cell, or a cell line derived therefrom, such as a 293T cell or a 293F cell.

5. The transformant according to claim 4, wherein the transformant further expresses granzyme and/or perforin; the amino acid sequence of the granzyme is as shown in SEQ ID NO: 10, and the amino acid sequence of the perforin is as shown in SEQ ID NO: 8;

    preferably, the granzyme and/or perforin is fused to CD63, wherein the amino acid sequence of the CD63 is as shown in SEQ ID NO: 6;
    more preferably, the transformant further overexpresses an exosome secretion-promoting protein, wherein the exosome secretion-promoting protein is CX43, KIBRA, or Rab27a; the NCBI reference sequence number for the amino acid sequence of the CX43 is NP_000156.1, preferably with S replaced by A at position 368; the NCBI reference sequence number for the amino acid sequence of the KIBRA is NP_056053.1; the NCBI reference sequence number for the amino acid sequence of the Rab27a is NP_899058.1;
    further preferably, the nucleotide sequence encoding the granzyme is as shown in SEQ ID NO: 9; the nucleotide sequence encoding the perforin is as shown in SEQ ID NO: 7; the nucleotide sequence encoding the CD63 is as shown in SEQ ID NO: 5; the NCBI reference sequence number for the nucleotide sequence encoding the CX43 is NM_000165.5, preferably with AGC replaced by GCC at positions 1317 to 1319; the NCBI reference sequence number for the nucleotide sequence encoding the KIBRA is XM_005265853.3; and/or the NCBI reference sequence number for the nucleotide sequence encoding the Rab27a is NM_183236.3.

6. The transformant according to claim 5, wherein the genes encoding the granzyme, perforin, CD63, KIBRA, and/or Rab27a are located in an expression plasmid;
preferably, the backbone of the expression plasmid is pcDNA3.1 or pCMV.

7. A method for preparing an exosome, wherein the method comprises culturing the transformant according to any one of claims 4 to 6, followed by isolation and purification to obtain the exosome from the culture medium of the transformant.

8. An exosome, wherein the exosome comprises the CAR molecule according to claim 1;
preferably, the exosome is prepared by the method according to claim 7.

9. A pharmaceutical composition, wherein the pharmaceutical composition comprises one or more components selected from the group consisting of: the CAR molecule according to claim 1, the transformant according to any one of claims 4 to 6, and the exosome according to claim 8;
preferably, the pharmaceutical composition further comprises a pharmaceutically acceptable carrier.

10. Use of the CAR molecule according to claim 1, the transformant according to any one of claims 4 to 6, the exosome according to claim 8, or the pharmaceutical composition according to claim 9 in the manufacture of a medicament for preventing and/or treating a tumor associated with CD19 and/or CD22 expression;

    preferably, the tumor is acute B lymphoblastic leukemia;
    more preferably, the acute B lymphoblastic leukemia is B-ALL leukemia or aggressive B-cell lymphoma.

FIG. 1

FIG. 2

PLVX-EF1ª--CD22
9199 bp

FIG. 3

FIG. 4

FIG. 5

FIG. 6

FIG. 7

Positive target cells

Negative target cells

FIG. 8

FIG. 9

FIG. 10

APC CD81

PE CD63

FIG. 11

FIG. 11 (Continued)

FIG. 12

FIG. 13

FIG. 14

FIG. 15

FIG. 16

FIG. 17

## INTERNATIONAL SEARCH REPORT

| International application No. |
| --- |
| **PCT/CN2024/072334** |

**A.    CLASSIFICATION OF SUBJECT MATTER**

C07K19/00(2006.01)i;  C12N15/86(2006.01)i;  A61P35/00(2006.01)i;  A61P15/02(2006.01)i

According to International Patent Classification (IPC) or to both national classification and IPC

**B.    FIELDS SEARCHED**

Minimum documentation searched (classification system followed by classification symbols)

IPC:C07K; C12N; A61P

Documentation searched other than minimum documentation to the extent that such documents are included in the fields searched

Electronic data base consulted during the international search (name of data base and, where practicable, search terms used)

CJFD, CNKI, CNABS, CNTXT, ENTXT, ENTXTC, VEN, BING, WEB OF SCIENCE, NCBI, EBI, 中国专利生物序列检索数据库, Chinese Patent Biological Sequence Retrieval System: 嵌合抗原受体, CAR, CD19, CD22, 颗粒酶, 穿孔素, CD63, CX43, KIBRA, Rab27a, 慢病毒, 载体, 外泌体, granzyme, perforin, lentivirus, exosome, SEQ ID NO: 1-10

**C.    DOCUMENTS CONSIDERED TO BE RELEVANT**

| Category* | Citation of document, with indication, where appropriate, of the relevant passages | Relevant to claim No. |
| --- | --- | --- |
| X | CN 113528581 A (SEATTLE CHILDREN'S HOSPITAL (DBA SEATTLE CHILDREN'S RESEARCH INSTITUTE)) 22 October 2021 (2021-10-22)<br>claims 1-25, and description, paragraphs 27, 98-116, 170-187, 279, and 309, table 2, and SEQ ID NO:10-11 | 1-4 |
| Y | CN 113528581 A (SEATTLE CHILDREN'S HOSPITAL (DBA SEATTLE CHILDREN'S RESEARCH INSTITUTE)) 22 October 2021 (2021-10-22)<br>claims 1-25, and description, paragraphs 27, 98-116, 170-187, 279, and 309, table 2, and SEQ ID NO:10-11 | 1-10 |
| Y | CN 110869046 A (CELLECTIS SA) 06 March 2020 (2020-03-06)<br>claims 1, 6-7, and 18-27, and description, paragraphs 176-177, 255-256, 685-710, and 885-890, table 1, and SEQ ID NO:23 | 1-10 |
| Y | CN 111465616 A (ABCLON INC.) 28 July 2020 (2020-07-28)<br>description, paragraphs 133-152, and SEQ ID NO:85 | 1-10 |

☑ Further documents are listed in the continuation of Box C.    ☑ See patent family annex.

| | |
| --- | --- |
| *    Special categories of cited documents:<br>"A"  document defining the general state of the art which is not considered to be of particular relevance<br>"D"  document cited by the applicant in the international application<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |

| Date of the actual completion of the international search | Date of mailing of the international search report |
| --- | --- |
| **19 March 2024** | **22 March 2024** |

| Name and mailing address of the ISA/CN | Authorized officer |
| --- | --- |
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

# EP 4 653 467 A1

## INTERNATIONAL SEARCH REPORT

| International application No. |
|---|
| **PCT/CN2024/072334** |

### C. DOCUMENTS CONSIDERED TO BE RELEVANT

| Category* | Citation of document, with indication, where appropriate, of the relevant passages | Relevant to claim No. |
|---|---|---|
| Y | CN 113444742 A (LUBAOTE (NANJING) ENVIRONMENTAL PROTECTION TECHNOLOGY CO., LTD.) 28 September 2021 (2021-09-28) abstract, and claims 1-9 | 5-10 |
| X | CN 109970862 A (GRACELL BIOTECHNOLOGY (SHANGHAI) CO., LTD.) 05 July 2019 (2019-07-05) claims 1-10, and description, paragraphs 5-10, specific embodiments, and SEQ ID NO:4 | 1-4 |
| X | CN 113122579 A (HEBEI SENLANG BIOTECHNOLOGY CO., LTD.) 16 July 2021 (2021-07-16) specific embodiments, and SEQ ID NO:1 | 1-4 |
| X | GenBank:QHQ73567.1. "FMC63-CD828Z[synthetic construct]" *GenBank*, 02 February 2020 (2020-02-02), entire document | 1-4 |
| Y | CN 103946242 A (THE UNITED STATES OF AMERICA, AS REPRESENTED BY THE SECRETARY, DEPARTMENT OF HEALTH & HUMAN SERVICES) 23 July 2014 (2014-07-23) claims 1-25, and description, paragraph 11, and SEQ ID NO:18, and SEQ ID NO:32 | 1-10 |
| A | CN 112272706 A (FLAGSHIP PIONEERING INNOVATIONS V, INC.) 26 January 2021 (2021-01-26) entire document | 1-10 |
| A | KOJIMA,R. et al. "Designer exosomes produced by implanted cells intracerebrally deliver therapeutic cargo for Parkinson's disease treatment." *Nature Communications*, Vol. 9, 31 December 2018 (2018-12-31), article no. 1305, pages 1-10 | 1-10 |
| A | KORELL,F. et al. "Understanding CAR T cell-tumor interactions: Paving the way for successful clinical outcomes." *Med*, Vol. 3, 12 August 2022 (2022-08-12), pages 538-564 | 1-10 |
| A | DAI,H. et al. "Bispecific CAR-T cells targeting both CD19 and CD22 for therapy of adults with relapsed or refractory B cell acute lymphoblastic leukemia." *Journal of Hematology & Oncology*, Vol. 13, 03 April 2020 (2020-04-03), article no. 30, pages 1-11 | 1-10 |

Form PCT/ISA/210 (second sheet) (July 2022)

44

**INTERNATIONAL SEARCH REPORT**

| International application No. |
| --- |
| **PCT/CN2024/072334** |

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

| INTERNATIONAL SEARCH REPORT<br>Information on patent family members | | International application No.<br>**PCT/CN2024/072334** |
|---|---|---|

| Patent document<br>cited in search report | Publication date<br>(day/month/year) | Patent family member(s) | Publication date<br>(day/month/year) |
|---|---|---|---|
| CN 113528581 A | 22 October 2021 | US 2022372140 A1 | 24 November 2022 |
| | | MX 2016013158 A | 27 April 2017 |
| | | MY 184163 A | 24 March 2021 |
| | | KR 20160143762 A | 14 December 2016 |
| | | KR 102463529 B1 | 07 November 2022 |
| | | ES 2867224 T3 | 20 October 2021 |
| | | WO 2015157399 A1 | 15 October 2015 |
| | | WO 2015157399 A9 | 24 November 2016 |
| | | AU 2021201679 A1 | 08 April 2021 |
| | | AU 2021201679 B2 | 04 May 2023 |
| | | MX 2016013160 A | 09 February 2017 |
| | | MY 185678 A | 30 May 2021 |
| | | SG 10201808833 XA | 29 November 2018 |
| | | KR 20230038310 A | 17 March 2023 |
| | | CA 2945302 A1 | 15 October 2015 |
| | | EP 3943507 A1 | 26 January 2022 |
| | | JP 2017513470 A | 01 June 2017 |
| | | JP 6765968 B2 | 07 October 2020 |
| | | JP 2017518037 A | 06 July 2017 |
| | | JP 6772068 B2 | 21 October 2020 |
| | | KR 20160144430 A | 16 December 2016 |
| | | KR 102387243 B1 | 14 April 2022 |
| | | KR 102387243 B9 | 10 July 2023 |
| | | KR 20220153100 A | 17 November 2022 |
| | | KR 102618955 B1 | 27 December 2023 |
| | | AU 2015243920 A1 | 03 November 2016 |
| | | AU 2015243920 B2 | 08 October 2020 |
| | | AU 2021273652 A1 | 16 December 2021 |
| | | KR 20160144432 A | 16 December 2016 |
| | | KR 102509481 B1 | 10 March 2023 |
| | | JP 2020195380 A | 10 December 2020 |
| | | JP 7062720 B2 | 06 May 2022 |
| | | SG 11201608395 PA | 29 November 2016 |
| | | JP 2017513520 A | 01 June 2017 |
| | | JP 6788573 B2 | 25 November 2020 |
| | | JP 6788573 B6 | 16 December 2020 |
| | | NZ 725079 A | 23 March 2018 |
| | | JP 2022184989 A | 13 December 2022 |
| | | AU 2015243922 A1 | 03 November 2016 |
| | | AU 2015243922 B2 | 05 August 2021 |
| | | AU 2021200007 A1 | 04 March 2021 |
| | | SG 11201608392 WA | 29 November 2016 |
| | | SG 11201608396 YA | 29 November 2016 |
| | | AU 2015243882 A1 | 03 November 2016 |
| | | AU 2015243882 B2 | 12 March 2020 |
| | | AU 2015243882 C1 | 24 September 2020 |
| | | US 2021002364 A1 | 07 January 2021 |
| | | US 11414486 B2 | 16 August 2022 |
| | | MX 2016013149 A | 27 April 2017 |
| | | IL 297591 A | 01 December 2022 |

Form PCT/ISA/210 (patent family annex) (July 2022)

## INTERNATIONAL SEARCH REPORT
### Information on patent family members

| International application No. |
|---|
| **PCT/CN2024/072334** |

| Patent document cited in search report | Publication date (day/month/year) | Patent family member(s) | | Publication date (day/month/year) |
|---|---|---|---|---|
| | | RU | 2016143381 A | 11 May 2018 |
| | | RU | 2016143381 A3 | 30 November 2018 |
| | | RU | 2752275 C2 | 26 July 2021 |
| | | EP | 3129399 A1 | 15 February 2017 |
| | | EP | 3129399 A4 | 08 November 2017 |
| | | EP | 3129399 B1 | 26 May 2021 |
| | | JP | 2021036867 A | 11 March 2021 |
| | | JP | 7106610 B2 | 26 July 2022 |
| | | JP | 2017515464 A | 15 June 2017 |
| | | SG | 10201808819 XA | 29 November 2018 |
| | | RU | 2016143385 A | 15 May 2018 |
| | | RU | 2016143385 A3 | 30 November 2018 |
| | | RU | 2751921 C2 | 20 July 2021 |
| | | WO | 2015157391 A1 | 15 October 2015 |
| | | US | 2021371517 A1 | 02 December 2021 |
| | | PH | 12016502010 A1 | 09 January 2017 |
| | | BR | 112016023523 A2 | 17 October 2017 |
| | | NZ | 758289 A | 26 January 2024 |
| | | NZ | 739448 A | 25 October 2019 |
| | | US | 2022380461 A1 | 01 December 2022 |
| | | US | 2017267742 A1 | 21 September 2017 |
| | | US | 10611837 B2 | 07 April 2020 |
| | | PH | 12016502012 A1 | 09 January 2017 |
| | | IL | 248229 A | 30 November 2016 |
| | | IL | 248229 B1 | 01 January 2023 |
| | | IL | 248229 B2 | 01 May 2023 |
| | | MY | 192522 A | 25 August 2022 |
| | | BR | 112016023500 A2 | 17 October 2017 |
| | | RU | 2016143389 A | 15 May 2018 |
| | | RU | 2016143389 A3 | 04 December 2018 |
| | | KR | 20220051024 A | 25 April 2022 |
| | | KR | 102600544 B1 | 09 November 2023 |
| | | WO | 2015157432 A1 | 15 October 2015 |
| | | CA | 2945305 A1 | 15 October 2015 |
| | | CA | 2945305 C | 17 October 2023 |
| | | JP | 2017512484 A | 25 May 2017 |
| | | JP | 6765967 B2 | 14 October 2020 |
| | | MY | 186846 A | 26 August 2021 |
| | | MX | 2016013159 A | 27 April 2017 |
| | | US | 2019248891 A1 | 15 August 2019 |
| | | US | 11155616 B2 | 26 October 2021 |
| | | US | 2018009891 A1 | 11 January 2018 |
| | | US | 10865242 B2 | 15 December 2020 |
| | | CA | 2945303 A1 | 15 October 2015 |
| | | CA | 2945303 C | 13 February 2024 |
| | | BR | 112016023517 A2 | 17 October 2017 |
| | | SG | 11201608393 TA | 29 November 2016 |
| | | KR | 20160138298 A | 02 December 2016 |
| | | KR | 102508166 B1 | 13 March 2023 |
| | | US | 2017015746 A1 | 19 January 2017 |

Form PCT/ISA/210 (patent family annex) (July 2022)

| INTERNATIONAL SEARCH REPORT | | International application No. | |
|---|---|---|---|
| Information on patent family members | | PCT/CN2024/072334 | |

| Patent document cited in search report | Publication date (day/month/year) | Patent family member(s) | | | Publication date (day/month/year) |
|---|---|---|---|---|---|
| | | JP | 2021019589 | A | 18 February 2021 |
| | | JP | 7093385 | B2 | 29 June 2022 |
| | | MX | 2022010807 | A | 27 September 2022 |
| | | WO | 2015157384 | A1 | 15 October 2015 |
| | | ES | 2880932 | T3 | 26 November 2021 |
| | | KR | 20160144431 | A | 16 December 2016 |
| | | US | 2017029774 | A1 | 02 February 2017 |
| | | CA | 2945320 | A1 | 15 October 2015 |
| | | EP | 3129053 | A1 | 15 February 2017 |
| | | EP | 3129053 | A4 | 08 November 2017 |
| | | JP | 2021000117 | A | 07 January 2021 |
| | | JP | 7148580 | B2 | 05 October 2022 |
| | | MX | 2016013144 | A | 27 April 2017 |
| | | BR | 112016023513 | A2 | 17 October 2017 |
| | | EP | 3954708 | A1 | 16 February 2022 |
| | | SA | 516380056 | B1 | 04 July 2021 |
| | | WO | 2015157386 | A1 | 15 October 2015 |
| | | EP | 3129480 | A1 | 15 February 2017 |
| | | EP | 3129480 | A4 | 16 August 2017 |
| | | IL | 248235 | A0 | 30 November 2016 |
| | | IL | 248235 | B | 31 January 2021 |
| | | EP | 4056201 | A1 | 14 September 2022 |
| | | IL | 248243 | A0 | 30 November 2016 |
| | | IL | 248243 | B1 | 01 December 2023 |
| | | BR | 112016023507 | A2 | 17 October 2017 |
| | | AU | 2015243927 | A1 | 03 November 2016 |
| | | AU | 2015243927 | B2 | 02 September 2021 |
| | | US | 2017152297 | A1 | 01 June 2017 |
| | | US | 10266592 | B2 | 23 April 2019 |
| | | RU | 2016143384 | A | 11 May 2018 |
| | | RU | 2016143384 | A3 | 30 November 2018 |
| | | RU | 2729112 | C2 | 04 August 2020 |
| | | US | 2022064292 | A1 | 03 March 2022 |
| | | US | 2021139583 | A1 | 13 May 2021 |
| | | EP | 3129471 | A1 | 15 February 2017 |
| | | EP | 3129471 | A4 | 08 November 2017 |
| | | JP | 2022109953 | A | 28 July 2022 |
| | | PH | 12016502009 | A1 | 09 January 2017 |
| | | IL | 248238 | A0 | 30 November 2016 |
| | | EP | 3129405 | A1 | 15 February 2017 |
| | | EP | 3129405 | A4 | 09 August 2017 |
| | | EP | 3129405 | B1 | 24 February 2021 |
| | | CA | 2945308 | A1 | 15 October 2015 |
| | | CA | 2945308 | C | 31 October 2023 |
| | | NZ | 725081 | A | 23 February 2018 |
| | | JP | 2022153456 | A | 12 October 2022 |
| | | JP | 7402933 | B2 | 21 December 2023 |
| | | PH | 12016502011 | A1 | 09 January 2017 |
| | | SG | 10201808825 | XA | 29 November 2018 |
| | | SG | 10201808811 | QA | 29 November 2018 |

Form PCT/ISA/210 (patent family annex) (July 2022)

**INTERNATIONAL SEARCH REPORT**
Information on patent family members

International application No.

**PCT/CN2024/072334**

| Patent document cited in search report | | | Publication date (day/month/year) | Patent family member(s) | | | Publication date (day/month/year) |
|---|---|---|---|---|---|---|---|
| | | | | AU | 2015243849 | A1 | 03 November 2016 |
| | | | | AU | 2015243849 | A9 | 25 July 2019 |
| | | | | AU | 2015243849 | B2 | 17 December 2020 |
| | | | | RU | 2016143388 | A | 14 May 2018 |
| | | | | RU | 2016143388 | A3 | 30 November 2018 |
| | | | | RU | 2751920 | C2 | 20 July 2021 |
| CN | 110869046 | A | 06 March 2020 | RU | 2019133365 | A | 30 April 2021 |
| | | | | RU | 2019133365 | A3 | 20 July 2021 |
| | | | | JP | 2020512019 | A | 23 April 2020 |
| | | | | JP | 7314115 | B2 | 25 July 2023 |
| | | | | IL | 269538 | A | 28 November 2019 |
| | | | | IL | 269538 | B1 | 01 March 2023 |
| | | | | IL | 269538 | B2 | 01 July 2023 |
| | | | | BR | 112019020203 | A2 | 02 June 2020 |
| | | | | EP | 3592380 | A1 | 15 January 2020 |
| | | | | AU | 2018246377 | A1 | 17 October 2019 |
| | | | | KR | 20200015467 | A | 12 February 2020 |
| | | | | MX | 2019011475 | A | 16 December 2019 |
| | | | | US | 2021161954 | A1 | 03 June 2021 |
| | | | | JP | 2023139070 | A | 03 October 2023 |
| | | | | CA | 3058268 | A1 | 04 October 2018 |
| | | | | US | 2021100839 | A1 | 08 April 2021 |
| | | | | US | 11690873 | B2 | 04 July 2023 |
| | | | | WO | 2018178377 | A1 | 04 October 2018 |
| | | | | EP | 3592379 | A1 | 15 January 2020 |
| | | | | AU | 2018246378 | A1 | 17 October 2019 |
| | | | | WO | 2018178378 | A1 | 04 October 2018 |
| CN | 111465616 | A | 28 July 2020 | US | 2023099646 | A1 | 30 March 2023 |
| | | | | EP | 3722313 | A2 | 14 October 2020 |
| | | | | EP | 3722313 | A4 | 24 February 2021 |
| | | | | AU | 2018379502 | A1 | 18 June 2020 |
| | | | | CA | 3083936 | A1 | 13 June 2019 |
| | | | | CA | 3083936 | C | 23 August 2022 |
| | | | | KR | 20190067125 | A | 14 June 2019 |
| | | | | KR | 102136063 | B1 | 22 July 2020 |
| | | | | US | 2020384023 | A1 | 10 December 2020 |
| | | | | US | 11534462 | B2 | 27 December 2022 |
| | | | | WO | 2019112347 | A2 | 13 June 2019 |
| | | | | WO | 2019112347 | A3 | 01 August 2019 |
| | | | | JP | 2021505137 | A | 18 February 2021 |
| | | | | JP | 7089806 | B2 | 23 June 2022 |
| CN | 113444742 | A | 28 September 2021 | None | | | |
| CN | 109970862 | A | 05 July 2019 | None | | | |
| CN | 113122579 | A | 16 July 2021 | None | | | |
| CN | 103946242 | A | 23 July 2014 | CA | 2851795 | A1 | 25 April 2013 |
| | | | | CA | 2851795 | C | 13 November 2018 |
| | | | | US | 2014274909 | A1 | 18 September 2014 |
| | | | | US | 9868774 | B2 | 16 January 2018 |
| | | | | IN | 2902014A | | 03 July 2015 |

Form PCT/ISA/210 (patent family annex) (July 2022)

**INTERNATIONAL SEARCH REPORT**
Information on patent family members

International application No.

**PCT/CN2024/072334**

| Patent document cited in search report | | | Publication date (day/month/year) | Patent family member(s) | | | Publication date (day/month/year) |
|---|---|---|---|---|---|---|---|
| | | | | BR | 112014008849 | A2 | 12 September 2017 |
| | | | | AU | 2017225049 | A1 | 28 September 2017 |
| | | | | AU | 2017225049 | B2 | 08 August 2019 |
| | | | | JP | 2017212996 | A | 07 December 2017 |
| | | | | ES | 2654060 | T3 | 12 February 2018 |
| | | | | EP | 2768863 | A1 | 27 August 2014 |
| | | | | EP | 2768863 | B1 | 27 September 2017 |
| | | | | RU | 2014118555 | A | 27 November 2015 |
| | | | | RU | 2644243 | C2 | 08 February 2018 |
| | | | | WO | 2013059593 | A1 | 25 April 2013 |
| | | | | JP | 2014534207 | A | 18 December 2014 |
| | | | | JP | 6267644 | B2 | 24 January 2018 |
| | | | | AU | 2012325915 | A1 | 24 April 2014 |
| CN | 112272706 | A | 26 January 2021 | IL | 276715 | A | 30 September 2020 |
| | | | | KR | 20200144093 | A | 28 December 2020 |
| | | | | CA | 3091478 | A1 | 22 August 2019 |
| | | | | SG | 11202007606 | QA | 29 September 2020 |
| | | | | JP | 2021513849 | A | 03 June 2021 |
| | | | | AU | 2019222560 | A1 | 27 August 2020 |
| | | | | MX | 2020008542 | A | 08 January 2021 |
| | | | | EP | 3752623 | A1 | 23 December 2020 |
| | | | | BR | 112020016570 | A2 | 15 December 2020 |
| | | | | WO | 2019161281 | A1 | 22 August 2019 |
| | | | | US | 2021137839 | A1 | 13 May 2021 |

Form PCT/ISA/210 (patent family annex) (July 2022)

**EP 4 653 467 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 2023100571558